# EUROPEAN PATENT APPLICATION

(11) **EP 4 847 632 A1**
(43) Date of publication of application: **02.12.2026**
(21) Application number: 25762321.5
(22) Date of filing: 11.02.2025
(51) Int. Cl.: C12Q 1/6886, A61K 45/06, A61P 35/00, G01N 33/68

(54) **TLK BIOMARKER COMPOSITION FOR PREDICTING PARP INHIBITOR TREATMENT RESPONSIVENESS OR PROGNOSIS OF BRCA MUTANT CANCER PATIENTS AND USE THEREOF**

(30) Priority: 26.02.2024 KR 20240027236
(71) Applicant: National Cancer Center, Goyang-si Gyeonggi-do 10408 (KR)
(72) Inventor: LEE, Kyung Yong, Seoul 06768 (KR)
(74) Representative: Fuchs Patentanwälte Partnerschaft mbB
(86) International application number: PCT/KR2025/001979
(87) International publication number: WO 2025/183387

(57) **Abstract**

The present invention relates to a TLK biomarker composition for predicting the treatment responsiveness to, or prognosis of, a PARP inhibitor in a BRCA mutant cancer patient, and to a use thereof. In the present invention, it was confirmed that, in cancer cells having a BRCA1 mutation, human TLK (Tousled-like Kinase) is essential for NHEJ-mediated DSB repair and PARP inhibitor sensitivity. It was confirmed that, although TLK is essential for the nuclear translocation of LC8 and for 53BP1 focus formation at DNA double-strand breaks (DSBs), upon TLK deficiency in BRCA1 mutant cancer, TLK promotes resistance to PARP inhibitors, decreases 53BP1 recruitment, and restores homologous recombination (HR). Therefore, the TLK biomarker of the present invention can be usefully applied to predicting the treatment responsiveness to, or prognosis of, a PARP inhibitor in BRCA mutant cancer.

## Description

### [Technical Field]

The present invention relates to a TLK biomarker composition for predicting the treatment responsiveness to, or prognosis of, a PARP inhibitor in a BRCA mutant cancer patient, and to a use thereof. More particularly, the present invention relates to a TLK biomarker composition for predicting the treatment responsiveness to, or prognosis of, a PARP inhibitor in a BRCA mutant cancer patient, and to a method of providing information for predicting PARP inhibitor treatment responsiveness or prognosis using the same.

### [Background Art]

A DNA double-strand break (DSB) is the most cytotoxic lesion among DNA damages, and improper DSB repair often leads to several human diseases including a phenotype resembling premature aging, tumor formation, and genomic instability (Helleday, T., et al., DNA Repair (Amst), 6:923-935, 2007). To repair a DSB, cells mainly utilize two pathways, namely nonhomologous end joining (NHEJ) and homologous recombination (HR) (Ceccaldi, R., et al., Trends Cell Biol., 26:52-64, 2015).

Because sister chromatids generated during DNA replication are required, HR occurs only at limited phases of the cell cycle (S to G2 phase), whereas NHEJ is a relatively rapid process that is activated throughout cell cycle progression, and most DSBs are repaired by NHEJ. The choice between NHEJ and HR at a DSB largely depends on whether the BRCA1-associated complex can access the DSB and resect the ends to generate ssDNA. A BRCA1 mutation generates homologous recombination deficiency (HRD), thereby increasing the lifetime risk of developing certain types of cancer, particularly breast cancer and ovarian cancer; conversely, HR deficiency in tumor cells renders the cancer vulnerable to treatment with poly(ADP-ribose) polymerase (PARP) inhibitors and platinum compounds (Toh, M. and Ngeow, J, Oncologist, 26:e1526-e1537, 2021).

A PARP inhibitor can inhibit base excision repair (BER), thereby inhibiting the repair of DNA damage in cancer cells and enhancing the efficacy of cancer therapies such as radiation, and has shown promising results in the treatment of breast cancer and ovarian cancer harboring BRCA mutations. PARP inhibitor-class therapeutics have already been launched on the market as new drugs, but follow-up research for indication expansion is active. To date, AstraZeneca's olaparib, Tesaro's niraparib, and Clovis Oncology's rucaparib, among others, have been utilized as ovarian cancer therapeutics, and olaparib has additionally been approved by the FDA for the indication of HER2-negative metastatic breast cancer with a BRCA mutation.

Although PARP inhibitors have shown significant benefits in progression-free survival and overall response rate, not all patients with a BRCA1 mutation respond to PARP inhibitor therapy.

According to a study published in the New England Journal of Medicine in 2019, the objective response rate of patients with BRCA1/2-mutated advanced breast cancer who received talazoparib, an FDA-approved PARP inhibitor, was 62.6%, and the median progression-free survival was 8.6 months. However, not all patients benefited from the treatment, and the study reported that 37.4% of the patients had progressive disease (Litton, J.K., et al., N Engl J Med, 379:753-763, 2018).

Likewise, according to a study published in the Journal of Clinical Oncology in 2020, among patients with BRCA1/2-mutated ovarian cancer who received niraparib, another FDA-approved PARP inhibitor, the objective response rate was 73% and the median progression-free survival was 12.9 months. However, the study reported that 27% of the patients did not respond to the treatment (Turner, N.C., et al., Clin Cancer Res, 27:5482-5491, 2021).

Therefore, despite the significant clinical benefits of PARP inhibitors for breast cancer and ovarian cancer patients harboring a BRCA1 mutation, there is a demand for the development of novel biomarkers for selecting patients to whom PARP inhibitor therapy is applicable and for predicting the treatment responsiveness to, or prognosis of, PARP inhibitor therapy.

Meanwhile, TLK (Tousled-like kinase) is a serine/threonine kinase family that is conserved across various eukaryotic organisms. In mammals, the two homologs TLK1 and TLK2 share a high level of sequence similarity and are expressed throughout the cell cycle. TLK plays a role in various cellular processes including DNA replication, chromatin remodeling, and the DNA damage response. TLK activity is highest during the S phase of the cell cycle, is essential for efficient DNA replication and proper chromosome segregation, and phosphorylates ASF1 to promote histone chaperone activity during DNA replication (Sillje, H.H., et al., EMBO J, 18, 5691-5702, 1999).

TLK is transiently inhibited by ATM-dependent Chk1 phosphorylation during the DNA damage response (DDR), but there are few reports emphasizing the role of TLK in the DNA damage response. TLK also plays a role in repairing DNA double-strand breaks, and depletion of TLK1 delays S-phase progression and impairs the DSB repair efficiency of cells recovering from irradiation (Sunavala-Dossabhoy, G., et al., BMC Mol Biol 6:19, 2005).

Overexpression of TLK1 has been shown to confer radioresistance to mouse mammary fibroblasts, and pharmacological inhibition of TLK using certain phenothiazines impairs DSB repair, resulting in the accumulation of yH2AX. Likewise, TLK has been associated with several cancers and developmental disorders (Khalil, M.I., et al., Cancer Drug Resist 5, 93-101, 2022).

### [Disclosure of the Invention]

### [Technical Problem]

In the present invention, as a result of efforts to select a biomarker capable of identifying the treatment responsiveness to, or the presence or absence of resistance to, a PARP inhibitor in a BRCA mutant cancer patient, the present inventors discovered a novel mechanism by which TLK promotes PARP inhibitor sensitivity by repairing DNA double-strand breaks (DSBs) in breast cancer and ovarian cancer cells having BRCA1 deficiency. In the present invention, it was confirmed that TLK1 and TLK2 promote NHEJ repair by regulating the nuclear translocation of LC8, which is required for the recruitment of 53BP1 to DSBs, and it was confirmed that when TLK1 or TLK2 is depleted, NHEJ is decreased and HR is increased despite BRCA1 deficiency, whereby triple-negative breast cancer (TNBC) cells and ovarian cancer cells having BRCA1 deficiency exhibit further increased resistance to PARP inhibitors.

Accordingly, an object of the present invention is to provide a TLK (Tousled-like kinase) biomarker composition for predicting the treatment responsiveness to, or prognosis of, a PARP inhibitor in a BRCA mutant cancer patient, a composition for predicting the treatment responsiveness to, or prognosis of, a PARP inhibitor comprising the same, and a kit for predicting the treatment responsiveness to, or prognosis of, a PARP inhibitor.

Another object of the present invention is to provide a method of providing information for predicting the treatment responsiveness to, or prognosis of, a PARP inhibitor using the biomarker composition.

Still another object of the present invention is to provide a composition for preventing or treating BRCA mutant cancer that exhibits resistance to a PARP inhibitor, comprising a substance for promoting the mRNA or protein expression of TLK (Tousled-like kinase), and a screening method thereof.

### [Technical Solution]

To achieve the above objects, the present invention provides a TLK (Tousled-like kinase) biomarker composition for predicting the treatment responsiveness to, or prognosis of, a PARP inhibitor in BRCA mutant cancer.

In a preferred embodiment of the present invention, the TLK may be TLK1 or TLK2.

In another preferred embodiment of the present invention, the PARP inhibitor may be olaparib, talazoparib, niraparib, rucaparib, veliparib, or pamiparib.

In addition, the present invention provides a composition for predicting the treatment responsiveness to, or prognosis of, a PARP inhibitor in BRCA mutant cancer, comprising an agent for measuring the mRNA or protein expression level of TLK (Tousled-like kinase), and a kit for predicting the treatment responsiveness to, or prognosis of, a PARP inhibitor in BRCA mutant cancer comprising the same.

In a preferred embodiment of the present invention, the TLK may be TLK1 or TLK2.

In another preferred embodiment of the present invention, the PARP inhibitor may be olaparib, talazoparib, niraparib, rucaparib, veliparib, or pamiparib.

In still another preferred embodiment of the present invention, the agent for measuring the mRNA expression level may be a sense and antisense primer, or a probe, that complementarily binds to the mRNA of TLK.

In still another preferred embodiment of the present invention, the agent for measuring the protein expression level may be an antibody, an interacting protein, a ligand, a nanoparticle, or an aptamer that specifically binds to a TLK protein or peptide fragment.

In still another preferred embodiment of the present invention, the BRCA mutant cancer may be BRCA mutant breast cancer, ovarian cancer, or uterine cancer.

To achieve another object, the present invention provides a method of providing information on the treatment responsiveness to, or prognosis of, a PARP inhibitor in BRCA mutant cancer, comprising the step of measuring the mRNA or protein expression level of TLK from a sample of a BRCA mutant cancer patient.

In a preferred embodiment of the present invention, when the mRNA or protein expression level of TLK is lower than that of a normal control, information may be provided indicating that the treatment responsiveness to the PARP inhibitor is low or that the prognosis is poor.

In addition, the present invention provides a method of providing information on the administration of a PARP inhibitor in BRCA mutant cancer, comprising the step of measuring the mRNA or protein expression level of TLK from a sample of a BRCA mutant cancer patient.

In a preferred embodiment of the present invention, when the mRNA or protein expression level of TLK is lower than that of a normal control, information may be provided indicating that the PARP inhibitor is not to be administered.

To achieve still another object, the present invention provides a pharmaceutical composition for preventing or treating BRCA mutant cancer that exhibits resistance to a PARP inhibitor, comprising a substance for promoting the mRNA or protein expression level of TLK.

In a preferred embodiment of the present invention, the composition may further comprise a PARP inhibitor, and the PARP inhibitor may be olaparib, talazoparib, niraparib, rucaparib, veliparib, or pamiparib.

In addition, the present invention provides a screening method for a substance for preventing or treating BRCA mutant cancer that exhibits resistance to a PARP inhibitor, comprising the steps of treating BRCA mutant cancer cells with a test substance and then measuring the mRNA or protein expression level of TLK.

In a preferred embodiment of the present invention, when the mRNA or protein expression level of TLK is increased by the test substance compared to a control, the test substance may be screened as a substance for preventing or treating BRCA mutant cancer that exhibits resistance to a PARP inhibitor.

### [Advantageous Effects of the Invention]

In the present invention, it was confirmed that, in cancer cells having a BRCA1 mutation, human TLK (Tousled-like Kinase) is essential for NHEJ-mediated DSB repair and PARP inhibitor sensitivity. It was confirmed that, although TLK does not depend on kinase activity, TLK1 is essential for the nuclear translocation of LC8 and for 53BP1 focus formation at DNA double-strand breaks (DSBs). In addition, it was confirmed that, upon TLK deficiency, TLK promotes resistance to PARP inhibitors in TNBC and OVCA cell lines having BRCA1 deficiency, decreases 53BP1 recruitment, and restores homologous recombination (HR). Therefore, the TLK biomarker of the present invention can be usefully applied to predicting the treatment responsiveness to, or prognosis of, a PARP inhibitor in BRCA mutant cancer.

### [Brief Description of the Drawings]

FIG. 1 shows the result that TLK promotes NHEJ-mediated DSB repair in a kinase-independent manner.
FIG. 1a is a schematic diagram of a GFP reporter assay method using U2OS EJ5-GFP to measure NHEJ efficiency.
FIG. 1b is immunoblot data confirming the expression levels of relevant proteins when cells were transfected with siRNA against TLK1 and/or TLK2, and FIG. 1c is the result of measuring the NHEJ efficiency thereof.
FIG. 1d is immunoblot data confirming the expression levels of relevant proteins in order to confirm the degree of restoration of NHEJ by siRNA-resistant wild-type TLK1 (WT) in cells in which endogenous TLK1 was depleted, and FIG. 1e is the result of measuring the NHEJ efficiency thereof.
FIG. 1f is the result of measuring NHEJ efficiency in order to confirm the degree of NHEJ restoration by transient expression of siRNA-resistant TLK2 WT in siTLK2-transfected cells.
FIG. 1g is data confirming the cellular sensitivity to a DSB inducer (Bleomycin) in TLK-depleted H1299 cells.
FIG. 1h is data confirming, by immunoblotting, the degree of delay in the disappearance of yH2AX upon bleomycin induction in TLK-depleted U2OS cells. In the figure, the amount of each protein was quantified using ImageJ software, and the ratio thereof was calculated and indicated.
FIG. 1i and FIG. 1j are data confirming that TLK promotes NHEJ regardless of kinase activity, in which cells stably expressing HA-TLK1-WT or HA-TLK1-KD (kinase-dead) were transfected with HA-I-Scel with or without siRNA TLK transfection, and then NHEJ efficiency was measured.
FIG. 1k is data confirming the degree of reduction in the sensitivity of H1299 cells to bleomycin upon expression of HA-TLK1-WT or HA-TLK1-KD.
FIG. 2 is data confirming that TLK is required for the DSB localization of 53BP1, an NHEJ-promoting factor.
FIG. 2a and FIG. 2b are an image (scale bar 10 µm) observing 53BP1 focus-positive cells when siRNA TLK-transfected A549 cells were treated with cisplatin, and data quantifying the same.
FIG. 2c and FIG. 2d are an image (scale bar 10 µm) observing RPA32 focus-positive cells when siRNA TLK-transfected A549 cells were treated with cisplatin, and data quantifying the same.
FIG. 2e is a schematic diagram showing U2OS ER-mCherry-Lacl-Fokl-DD cells for monitoring the recruitment of a protein of interest at an artificial DSB cleaved by Fokl endonuclease. The translated ER-mCherry-Lacl-Fokl-DD is stabilized by co-treatment with Shield-1 and 4-OHT, and when translocated into the nucleus, it generates DSBs at LacO repeat sites.
FIG. 2f and FIG. 2g are data confirming that the localization of 53BP1 at the Fokl cleavage site is decreased upon depletion of TLK1 or TLK2, in which U2OS ER-mCherry-Lacl-Fokl-DD cells were transfected with siRNA TLK, and then 53BP1 focus-positive cells were observed in an image, with data quantifying the same.
FIG. 3 is data confirming the promoting effect of TLK on the DSB localization of LC8 and its interaction with 53BP1.
FIG. 3a is an image (scale bar 10 µm) observing the DSB localization of Flag-LC8 when Flag-LC8-expressing U2OS cells were treated with bleomycin.
FIG. 3b to FIG. 3d are an image (scale bar 10 µm) observing Flag-LC8 and yH2AX focus-forming cells when U2OS cells stably expressing Flag-LC8 were transfected with siRNA TLK and then treated with bleomycin, and data quantifying the same. It was confirmed that Flag-LC8 focus formation at DSBs is decreased due to depletion of TLK1 or TLK2.
FIG. 3e is data observing, by immunoblotting, the degree of reduction of chromatin-bound LC8 according to TLK depletion when U2OS cells stably expressing Flag-LC8 were transfected with siRNA TLK and then treated with bleomycin. The immunoblots of γH2AX and histone H3 indicate the DNA damage response induced by bleomycin and chromatin enrichment of the extract, respectively; TCE denotes total cell extract, and Chr denotes the chromatin-enriched fraction.
FIG. 3f is data confirming the restoration of LC8 localization at the Fokl cleavage site by siRNA-resistant TLK1 in TLK1-depleted cells, in which a qChIP analysis was performed on U2OS ER-mCherry-Lacl-Fokl-DD cells using primers near the Fokl cut.
FIG. 3g is data confirming the degree of reduction in the interaction between 53BP1 and LC8 upon TLK1 or TLK2 deficiency, in which H1299 cells were transfected with the indicated siRNA TLK, treated with bleomycin, and then subjected to immunoblotting.
FIG. 4 is data confirming that the nuclear localization signal (NLS) of TLK is essential for the nuclear translocation of LC8 and NHEJ.
FIG. 4a is data of immunoblotting performed when U2OS cells were transfected with siRNA TLK and then treated with bleomycin. Cyt is the cytoplasmic fraction; Nuc is the nucleosol fraction; the immunoblots of NBS1 and GAPDH were used as representative markers for the nucleosol and cytoplasmic fractions, respectively, and it was confirmed that nuclear LC8 is decreased in TLK-depleted cells.
FIG. 4b is an image showing the highly conserved NLS sequences of TLK1 and TLK2 across various species. The putative NLS sequence was predicted using the cNLS Mapper program available at http//nls-mapper.iab.keio.ac.jp.
FIG. 4c confirms the failure of nuclear localization of the HA-TLK1 NLS mutant, showing a schematic diagram of the functional motifs of TLK1 including the NLS (upper panel), and an image observing the cells when amino acids that inactivate the NLS function of TLK1 and the cytoplasmic localization of HA-TLK1 NLSm were substituted (lower panel).
FIG. 4d is data of immunoprecipitation (IP) performed when HA-TLK1-WT or NLSm was overexpressed in HEK293T cells, showing that the interaction between TLK1 and LC8 is not affected by the NLS mutation.
FIG. 4e to FIG. 4g are immunoblot data according to cell fractionation when U2OS EJ5 reporter cells stably expressing siRNA-resistant HA-TLK1-WT, HA-TLK1-KD, or -NLSm were transfected with siRNA TLK and then treated with bleomycin, in which it was confirmed that restoration of the nuclear localization of LC8 and NHEJ failed due to the TLK1 NLS mutation. In the figure, the Nuc:Cyt ratio is indicated at the bottom of FIG. 4e, and the NHEJ efficiency and the quantification of the corresponding immunoblot are shown in FIG. 4f and FIG. 4g, respectively.
FIG. 4h is immunoblot data according to cell fractionation when U2OS EJ5 reporter cells stably expressing siRNA-resistant HA-TLK1-WT or HA-TLK1-KD (kinase-dead) were transfected with siRNA TLK and then treated with bleomycin, in which it was confirmed that the role of TLK in regulating the nuclear translocation of LC8 is functionally independent of its kinase activity.
FIG. 4i is data confirming the effect of ectopic expression of HA-TLK1 NLSm on the cellular sensitivity to bleomycin upon TLK1 depletion when H1299 cells stably expressing siTLK1-resistant WT or NLSm were transfected with siRNA TLK and then treated with bleomycin.
FIG. 4j is a schematic diagram showing the functional role of TLK in mediating the nuclear localization of LC8 to promote 53BP1-dependent NHEJ repair at DSBs.
FIG. 5 is data confirming that resistance to PARP inhibitors in BRCA1 mutant cancer is increased by TLK deficiency.
FIG. 5a and FIG. 5b are immunoblot data according to siRNA TLK transfection in BRCA1-proficient MDA-MB-468 cells, and data confirming cell viability in order to observe the sensitivity to a PARP inhibitor (Olaparib) according to TLK loss.
FIG. 5c to FIG. 5e are immunoblot data according to siRNA TLK transfection in BRCA1-mutant MDA-MB-436 cells, and data confirming cell viability in order to observe the presence or absence of resistance to PARP inhibitors (Olaparib and Veliparib) according to TLK loss.
FIG. 5f and FIG. 5g are immunoblot data according to siRNA TLK transfection in siBRCA1-transfected BRCA1-proficient MDA-MB-468 cells, and data confirming cell viability in order to observe the presence or absence of resistance to a PARP inhibitor (Olaparib) according to TLK loss.
FIG. 5h and FIG. 5i are immunoblot data according to siRNA TLK transfection in BRCA1-mutant UWB1.289 ovarian cancer cells, and data confirming cell viability in order to observe the presence or absence of resistance to a PARP inhibitor (Olaparib) according to TLK loss.
FIG. 6 is data confirming that HR is restored in BRCA1-deficient cells by TLK loss.
FIG. 6a is a schematic diagram illustrating the measurement of HR recovery according to BRCA1 depletion in U2OS DR-GFP cells.
FIG. 6b and FIG. 6c are data confirming, by immunoblotting, the expression level of each protein when U2OS DR-GFP cells were transfected with siBRCA1 and siRNA TLK, and data confirming the HR efficiency.
FIG. 6d and FIG. 6e are an image (scale bar 10 µm) observing 53BP1 focus-positive cells when A549 cells were transfected with siBRCA1 and siRNA TLK and then treated with bleomycin, and data quantifying the same.
FIG. 6f to FIG. 6h are an image (scale bar 10 µm) observing 53BP1 focus-positive cells when A549 cells were transfected with siBRCA1 and siRNA TLK and then treated with cisplatin, and data quantifying the same. In the absence of BRCA1, 53BP1 focus formation at DSBs was decreased when TLK1 and TLK2 were simultaneously depleted.
FIG. 6i and FIG. 6j are data confirming, by immunoblotting, the expression level of each protein in order to analyze NHEJ efficiency when NHEJ/DsRed293B cells were transfected with siBRCA1 and siRNA TLK, and data analyzing NHEJ efficiency by counting the number of DsRed-positive cells in FACS analysis.
FIG. 6k is data of HR analysis performed using the method of FIG. 6a in order to confirm whether TLK and LC8 act in the same DSB repair pathway upon BRCA1 inactivation, in which U2OS DR-GFP cells were transfected with siBRCA1, siLC8, or siRNA TLK, and then the HR efficiency was analyzed.
FIG. 7 is data observing the prognosis according to the loss of TLK in BRCA1-deficient patients.
FIG. 7a to FIG. 7c are patient survival plots generated by comparing patient groups with high expression of TLK1 or TLK2 with patient groups with low expression. Breast invasive carcinoma (BRCA), ovarian serous cystadenocarcinoma (OVCA), and uterine corpus endometrial carcinoma (UCEC) were selected from the TCGA database and classified based on the corresponding TLK expression of BRCA1 WT or deficient patients.
FIG. 7d is data analyzing the degree of risk in pan-cancer of patient cohorts with low expression of TLK1 or TLK2. The Cox coefficients of the low TLK1/TLK2 cohorts were analyzed across multiple cancers, where red indicates a higher risk (poor prognosis) for the group with low expression of TLK1 or TLK2, and blue indicates a lower risk (good prognosis). The patient groups were classified by the presence or absence of a BRCA1 mutation, and then classified by TLK expression level.
FIG. 7e is a box plot of TLK1 or TLK2 expression (z-score) in the BRCA1 WT and deficient cohorts.
FIG. 7f is a schematic diagram showing the proposed model for the role of TLK in NHEJ and PARP inhibitor sensitivity under BRCA mutant conditions.

### [Best Mode for Carrying Out the Invention]

Hereinafter, the present invention will be described in detail.

### Biomarker Composition for Predicting Treatment Responsiveness or Prognosis of PARP Inhibitor in BRCA Mutant Cancer

In one aspect, the present invention relates to a TLK (Tousled-like kinase) biomarker composition for predicting the treatment responsiveness to, or prognosis of, a PARP inhibitor in BRCA mutant cancer.

In the present invention, the TLK may be TLK1 or TLK2.

In the present invention, the PARP inhibitor may be olaparib, talazoparib, niraparib, rucaparib, veliparib, or pamiparib, but is not limited thereto, and any substance having a PARP-inhibitory effect known in the art, such as a next-generation PARP inhibitor that obtains clinical FDA or EMA approval, may be applied without limitation.

In a specific embodiment of the present invention, it was confirmed that TLK promotes 53BP1-dependent NHEJ activity, and that the NLS (nuclear localization signal) of TLK is essential for the nuclear translocation of LC8 and for 53BP1 focus formation at DSBs, rather than for kinase activity.

In BRCA1 mutant cancer, NHEJ activity was shown to be decreased due to TLK depletion. In addition, it was confirmed that, due to TLK depletion, 53BP1 recruitment is decreased and homologous recombination (HR) is restored, thereby promoting resistance to PARP inhibitors.

Therefore, the TLK biomarker of the present invention can be usefully applied to predicting the treatment responsiveness to, or prognosis of, a PARP inhibitor in BRCA mutant cancer.

### Composition for Predicting Treatment Responsiveness or Prognosis of PARP Inhibitor in BRCA Mutant Cancer

In another aspect, the present invention relates to a composition for predicting the treatment responsiveness to, or prognosis of, a PARP inhibitor in BRCA mutant cancer, comprising an agent for measuring the mRNA or protein expression level of the TLK (Tousled-like kinase).

The composition for predicting the treatment responsiveness to, or prognosis of, a PARP inhibitor in BRCA mutant cancer according to the present invention applies mutatis mutandis the above-described <Biomarker Composition>.

In the present invention, the BRCA mutant cancer may be breast cancer, ovarian cancer, or uterine cancer in which a BRCA mutation is present.

In the present invention, the agent for measuring the mRNA expression level may be a sense and antisense primer, or a probe, that complementarily binds to the mRNA of TLK.

Measurement of the mRNA expression level is a process of confirming the presence or absence and the degree of expression of the mRNA of a biomarker in a biological sample, in which the amount of mRNA is measured. Analysis methods therefor include, but are not limited to, reverse transcription polymerase chain reaction (RT-PCR), competitive RT-PCR, real-time RT-PCR, RNase protection assay (RPA), Northern blotting, and DNA chip.

The agent for measuring the mRNA level of the composite marker is characterized by being a primer pair, a probe, or an antisense nucleotide that specifically binds to the gene of the composite marker; and since the nucleic acid information of the genes is known in GeneBank and the like, a person skilled in the art can design these primer pairs, probes, or antisense nucleotides based on the sequences.

As used herein, the term "primer" refers to a fragment that recognizes a target gene sequence, including forward and reverse primer pairs, and is preferably a primer pair that provides analysis results having specificity and sensitivity.

As used herein, the term "probe" refers to a substance capable of specifically binding to a target substance to be detected in a sample, and refers to a substance through which the presence of the target substance in the sample can be specifically confirmed by said binding. The type of probe is not limited as long as it is a substance commonly used in the art, but is preferably PNA (peptide nucleic acid), LNA (locked nucleic acid), a peptide, a polypeptide, a protein, RNA, or DNA, and most preferably PNA.

As used herein, the term "antisense" refers to an oligomer having a sequence of nucleotide bases and an intersubunit backbone that allow the antisense oligomer to hybridize, by Watson-Crick base pairing, with a target sequence in RNA, thereby typically permitting the formation of an mRNA:oligomer heteroduplex within the target sequence. The oligomer may have exact sequence complementarity or near complementarity to the target sequence.

In the present invention, the agent for measuring the protein expression level may be an antibody, an interacting protein, a ligand, a nanoparticle, or an aptamer that specifically binds to the TLK protein or peptide fragment.

Measurement of the protein expression level is a process of confirming the presence or absence and the degree of expression of a protein expressed from a biomarker in a biological sample. The amount of protein can be confirmed using an antibody, an interacting protein, a ligand, a nanoparticle, or an aptamer that specifically binds to the protein or peptide fragment of the gene.

Methods for measuring or comparatively analyzing the protein expression level include, but are not limited to, protein chip analysis, immunoassay, ligand-binding assay, MALDI-TOF (Matrix-Assisted Laser Desorption/Ionization Time-of-Flight Mass Spectrometry) analysis, SELDI-TOF (Surface-Enhanced Laser Desorption/Ionization Time-of-Flight Mass Spectrometry) analysis, radioimmunoassay, radioimmunodiffusion, Ouchterlony immunodiffusion, rocket immunoelectrophoresis, tissue immunostaining, complement fixation assay, two-dimensional electrophoresis analysis, liquid chromatography-mass spectrometry (LC-MS), LC-MS/MS (liquid chromatography-mass spectrometry/mass spectrometry), Western blot, and ELISA (enzyme-linked immunosorbent assay).

As used herein, the term "antibody" refers to a substance that specifically binds to an antigen to cause an antigen-antibody reaction. For the purposes of the present invention, an antibody refers to an antibody that specifically binds to the biomarker of the present invention. The antibody of the present invention includes polyclonal antibodies, monoclonal antibodies, and recombinant antibodies.

### Kit for Predicting Treatment Responsiveness or Prognosis of PARP Inhibitor in BRCA Mutant Cancer

In still another aspect, the present invention relates to a kit for predicting the treatment responsiveness to, or prognosis of, a PARP inhibitor in BRCA mutant cancer, comprising a composition for predicting the treatment responsiveness to, or prognosis of, a PARP inhibitor in BRCA mutant cancer.

The kit for predicting the treatment responsiveness to, or prognosis of, a PARP inhibitor in BRCA mutant cancer according to the present invention applies mutatis mutandis the above-described <Biomarker Composition> or <Composition for Predicting Treatment Responsiveness or Prognosis of PARP Inhibitor>.

The kit can be prepared by a conventional preparation method known in the art. The kit may include, for example, an antibody in a lyophilized form, together with a buffer, a stabilizer, an inert protein, and the like.

The kit may further comprise a detectable label. The term "detectable label" refers to an atom or molecule that allows a molecule containing the label to be specifically detected among molecules of the same kind that do not contain the label. The detectable label may be attached to an antibody, an interacting protein, a ligand, a nanoparticle, or an aptamer that specifically binds to the protein or a fragment thereof. The detectable label may include a radionuclide, a fluorophore, or an enzyme.

The kit can be used according to various immunoassays or immunostaining methods known in the art. The immunoassays or immunostaining methods may include radioimmunoassay, radioimmunoprecipitation, immunoprecipitation, ELISA, capture-ELISA, inhibition or competition assay, sandwich assay, flow cytometry, immunofluorescence staining, and immunoaffinity purification. Preferably, the kit may be an RT-PCR (reverse transcription polymerase chain reaction) kit, a DNA chip kit, an ELISA (enzyme-linked immunosorbent assay) kit, a protein chip kit, a rapid kit, or an MRM (multiple reaction monitoring).

### Method of Providing Information on Treatment Responsiveness or Prognosis of PARP Inhibitor in BRCA Mutant Cancer

In still another aspect, the present invention relates to a method of providing information on the treatment responsiveness to, or prognosis of, a PARP inhibitor in BRCA mutant cancer, comprising the step of measuring the mRNA or protein expression level of TLK from a sample of a BRCA mutant cancer patient.

In the present invention, when the mRNA or protein expression level of TLK is lower than that of a normal control, information may be provided indicating that the treatment responsiveness to the PARP inhibitor is low or that the prognosis is poor.

In still another aspect, the present invention relates to a method of providing information on the administration of a PARP inhibitor in BRCA mutant cancer, comprising the step of measuring the mRNA or protein expression level of TLK from a sample of a BRCA mutant cancer patient.

In the present invention, when the mRNA or protein expression level of TLK is lower than that of a normal control, information may be provided indicating that the PARP inhibitor is not to be administered.

In the method, the term "biological sample" refers to a sample such as tissue, cells, blood, serum, plasma, saliva, cerebrospinal fluid, or urine, and preferably refers to blood, plasma, or serum.

In the information providing method, the method of measuring the mRNA expression level of the biomarker or the protein expression level thereof is as described above.

### Composition for Preventing or Treating BRCA Mutant Cancer Exhibiting Resistance to PARP Inhibitor and Screening Method Thereof

In still another aspect, the present invention relates to a pharmaceutical composition for preventing or treating BRCA mutant cancer that exhibits resistance to a PARP inhibitor, comprising a substance for promoting the mRNA or protein expression level of TLK.

The composition can be administered in combination with a PARP inhibitor, and the PARP inhibitor may be olaparib, talazoparib, niraparib, rucaparib, veliparib, or pamiparib.

In still another aspect, the present invention relates to a screening method for a substance for preventing or treating BRCA mutant cancer that exhibits resistance to a PARP inhibitor, comprising the steps of treating BRCA mutant cancer cells with a test substance and then measuring the mRNA or protein expression level of TLK.

In the present invention, when the mRNA or protein expression level of TLK is increased by the test substance compared to a control, the test substance may be screened as a substance for preventing or treating BRCA mutant cancer that exhibits resistance to a PARP inhibitor.

Hereinafter, the present invention will be described in more detail through Examples.

These Examples are only for illustrating the present invention, and it will be apparent to those of ordinary skill in the art that the scope of the present invention is not to be construed as being limited by these Examples.

### Example 1: Confirmation of the Efficacy of TLK in Promoting NHEJ-Mediated DSB Repair

### 1-1: Investigation of NHEJ Activity According to TLK Depletion

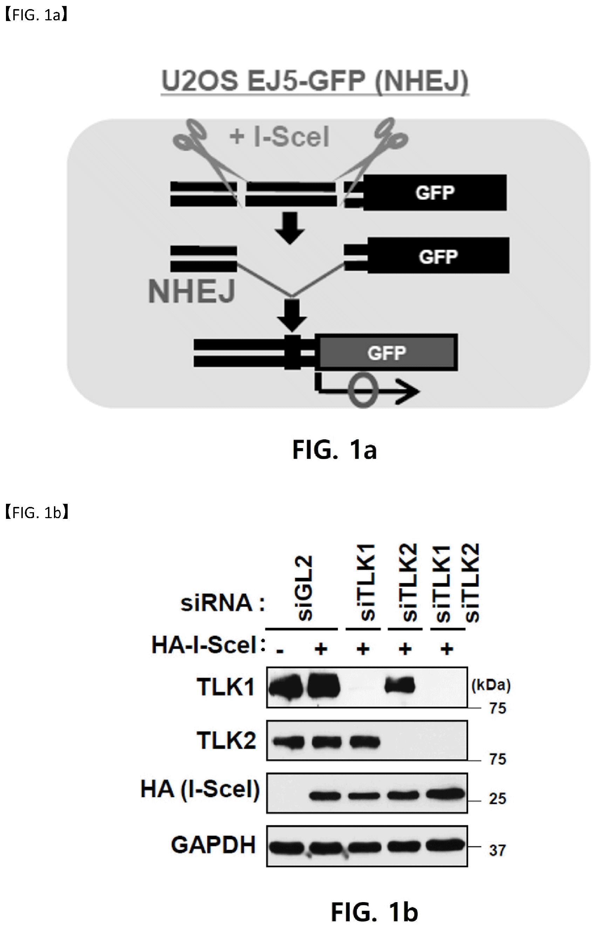

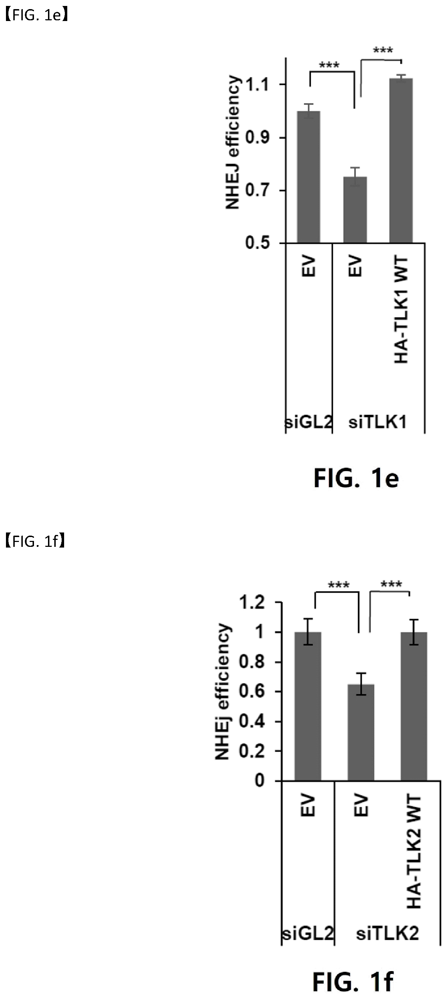

In the present invention, in order to investigate the unknown function of TLK in the repair of DNA double-strand breaks (DSBs) induced by the DNA damage response (DDR), an NHEJ reporter assay was performed using the U2OS EJ5-GFP cell line. As shown in FIG. 1a, in the U2OS EJ5-GFP cell line, intact GFP is generated when the I-Scel cleavage site of a mutant GFP gene is successfully repaired by intracellular NHEJ.

The U2OS EJ5-GFP cell line was prepared by a previously known method (Gunn, A., and Stark, J.M., Methods Mol Biol, 920:379-391, 2012) and cultured at 37°C using DMEM (Dulbecco's Modified Eagle's Medium, Gibco) medium containing 10% FBS and penicillin/streptomycin (1%, Thermo Scientific).

Subsequently, 3 × 10⁵ cells were seeded in a 6-well plate, siRNA was used for the depletion of TLK1 or TLK2, and the cells were transfected using Lipofectamine RNAiMAX for siRNA (13778150, Invitrogen) according to the manufacturer's instructions.
siGL2: 5'-CGUACGCGGAAUACUUCGA-3' (SEQ ID NO: 1)
siTLK1: 5'-GAAGCUCGGUCUAUUGUAA-3' (SEQ ID NO: 2)
siTLK2: 5'-GAUAGAAAGACAACGGAAA-3' (SEQ ID NO: 3)
siBRCA1: 5'-CCUGUCUCCACAAAGUGUG-3' (SEQ ID NO: 4)
siLC8: 5'-AGCCUAAAUUCCAAAUAC-3' (SEQ ID NO: 5)

After 24 hours, 1.5 or 2 µg of the I-Scel expression vector pCβASce was transfected using Lipofectamine 2000 (Invitrogen). The cells were harvested 72 hours after siRNA transfection and used for subsequent analysis experiments.

For immunoblotting, the U2OS EJ5-GFP cells transfected with the siRNA were lysed with RIPA lysis buffer (GenDEPOT) containing protease inhibitors for 15 minutes on ice and then briefly sonicated. About 30 to 50 µg of the lysate was loaded onto an SDS-PAGE gel and transferred to a nitrocellulose membrane (Amersham Protran). Thereafter, the membrane was blocked with 3% BSA in TBS-T (0.1% Tween 20 in TBS) at room temperature for 30 minutes and reacted with a primary antibody overnight at 4°C or for 1 hour at room temperature. The primary antibody was detected with a secondary antibody for 1 hour at room temperature and visualized using ECL solution (Millipore). The primary antibodies used in the present invention are as follows: TLK1 (4125S, Cell Signaling Technology), TLK2 (sc-393506, Santacruz), HA (sc-7392, Santacruz), GAPDH (6004-1-lg, Proteintech). Then, the ratio of HA-I-Scel to GAPDH was quantified from the immunoblot and normalized to the percentage of GFP-positive cells in each sample.

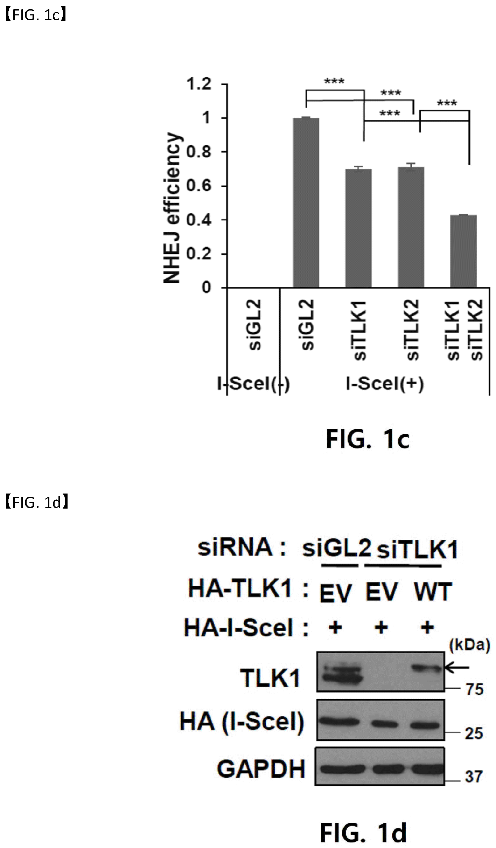

As a result, depletion of TLK1 or TLK2 decreased NHEJ, but co-depletion of TLK1 and TLK2 caused a further decrease in NHEJ compared to single depletion, confirming that TLK1 and TLK2 play redundant roles in promoting NHEJ repair at DSBs. In addition, the NHEJ efficiency was decreased by TLK depletion without a decrease in the expression of I-Scel, a marker of the DSB cleaved in the GFP cassette (FIG. 1b and FIG. 1c).

### 1-2: Investigation of NHEJ Activity According to Restoration of TLK Depletion

For restoration of TLK depletion, the TLK1 or TLK2 sequence was PCR-amplified and cloned into the pcDNA3-HA vector, and the siRNA-resistant TLK1 or TLK2 wild-type (WT) and kinase-dead (KD) constructs were generated using the following primers:
<siRNA-resistant TLK1-wild type; HA-TLK1-WT>
   F: 5'-CAAGTTAATGTCAGAGAAAGAGGCCAGGTCTATTGTAATGCAGATTG-3' (SEQ ID NO: 6)
   R: 5'-CAATCTGCATTACAATAGACCTGGCCTCTTTCTCTGACATTAACTTG-3' (SEQ ID NO: 7)
<siRNA-resistant TLK2-wild type; HA-TLK2-WT>
   F: 5'-CAGAGGGAAGAGATCGAAAGGCAACGGAAAATGTTAGCAAAGCGG-3' (SEQ ID NO: 8)
   R: 5'-CCGCTTTGCTAACATTTTCCGTTGCCTTTCGATCTCTTCCTCTG-3' (SEQ ID NO: 9)
<TLK1 KD; HA-TLK1-KD>
   F: 5'-GATTTCCAAC AAGGTTGCTGTATGGTCGGTTGGAG-3' (SEQ ID NO: 10)
   R: 5'-CTCCAACCGACCATACAGCAACCTTGTTGGAAATC-3' (SEQ ID NO: 11)

To generate stable cell lines, the plasmids were transfected into U2OS or U2OS EJ5 cells using Lipofectamine 2000 (11668019, Invitrogen), and 48 hours after transfection, the cells were treated with 500 µg/mL G418 (Invivogen) for at least one week for drug selection.

As a result, the NHEJ that was decreased upon depletion of TLK1 or TLK2 was completely restored by the siRNA-resistant version of wild-type TLK1 or TLK2, respectively, confirming that the decrease in NHEJ upon TLK loss was not due to off-target activity of the siRNA (FIG. 1d to FIG. 1f).

### 1-3: Confirmation of Cellular Sensitivity to a DSB Inducer in TLK-Depleted Cells

The cellular sensitivity was confirmed when TLK-depleted cells were treated with bleomycin, a DSB inducer. Transfection of H1299 cells or U2OS cells was performed by the same method as in <Example 1-1> and <Example 1-2>, and then the cells were treated with 2 µg/mL bleomycin (13877, Cayman Chemical) for 96 hours. Thereafter, immunoblotting, NHEJ activity, and cell viability were measured.

Cell viability analysis was measured using the Cell Titer 96 AQueous One Solution Cell Proliferation Assay kit (G3581, Promega) according to the manufacturer's instructions. Briefly, each cell transfected with siRNA was treated with bleomycin, and then a 1:5 mixture of Cell Titer 96 aqueous reagent and medium was added to the plate, and readings were obtained using a BioTek Power Wave HT 340 microplate reader. Viability analysis was normalized to the value of the untreated control.

In the immunoblot, H2AX and yH2AX (pS139-H2AX) were used as primary antibodies, and the amount of each protein was quantified using ImageJ software and the ratio thereof was calculated.

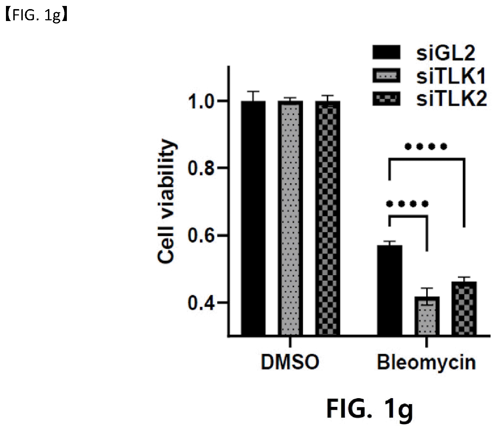

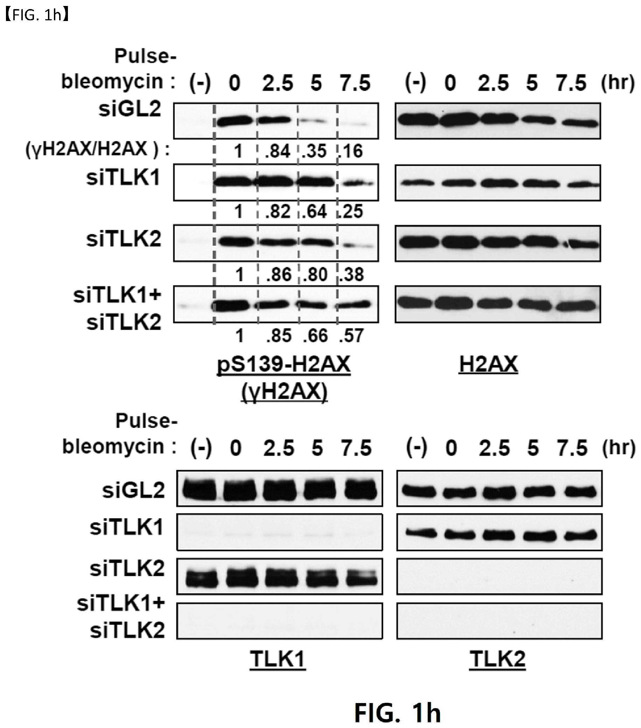

A defect in NHEJ can sensitize cells to DSBs, and indeed, depletion of TLK1 or TLK2 was shown to make cells more sensitive to bleomycin, a DSB inducer (FIG. 1g). After pulse treatment with bleomycin, γH2AX (phospho-H2AX) decreases over time. The disappearance of γH2AX was delayed by about 2.5 hours due to depletion of TLK1 or TLK2, and this delay was further increased by co-depletion of TLK1 and TLK2. This means that the two proteins are independently required for NHEJ (FIG. 1h).

### 1-4: Kinase-Independent Regulation of NHEJ Activity by TLK

To investigate the requirement for the kinase activity of TLK, a U2OS cell line stably expressing an HA-tagged TLK1 kinase-dead mutant (D608A) was generated, and NHEJ and cell survival analyses were performed.

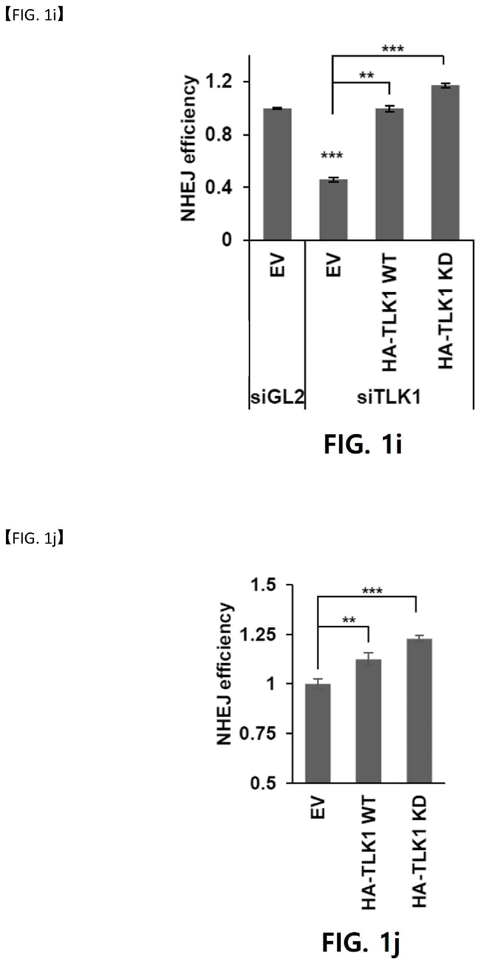

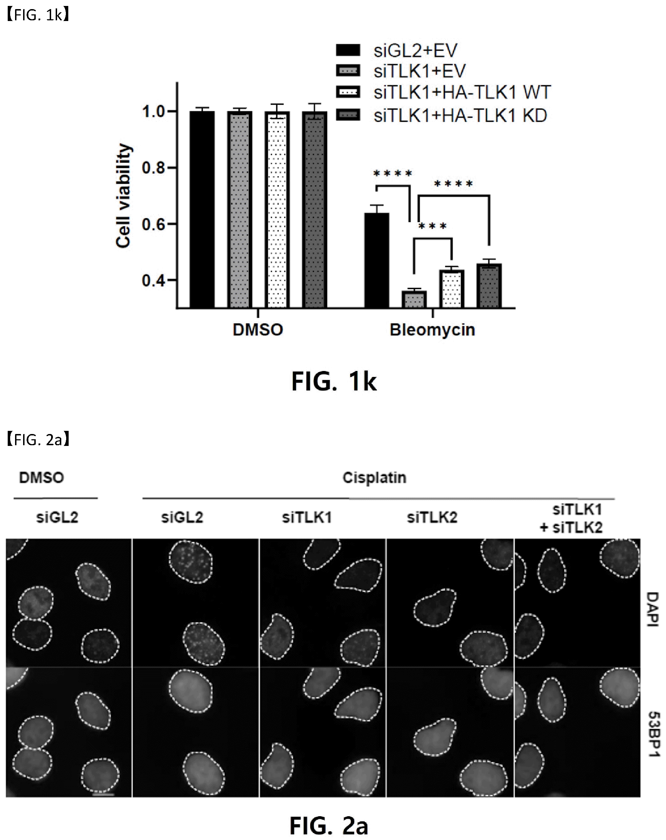

As a result, TLK1 KD (kinase-dead) was able to restore the NHEJ activity decreased by TLK depletion, and overexpression also increased NHEJ activity similarly to WT (FIG. 1i and FIG. 1j). In addition, upon TLK1 depletion, the bleomycin-induced cellular sensitivity was alleviated by ectopic expression of TLK1 WT (FIG. 1k) but was not alleviated in TLK1 KD (FIG. 1k).

Therefore, it was confirmed that TLK1 and TLK2 play independent roles in promoting NHEJ repair at DSBs and suppressing cellular sensitivity to DSBs, and that, at least in the case of TLK1, kinase activity is not required for the induction of NHEJ activity.

### Example 2: Confirmation of the Degree of DSB Localization of 53BP1 According to TLK Depletion

At a DNA double-strand break (DSB), the DSB localization of 53BP1 promotes NHEJ by antagonizing ssDNA formation at DSBs coated with RPA and Rad51 (Xu, Y., and Xu, D., Essays Biochem, 64:765-724, 2020). Therefore, in the present invention, the DSB localization of 53BP1, an NHEJ-promoting factor, was confirmed by immunostaining in TLK-depleted cells.

First, siRNA TLK-transfected A549 cells were plated on glass coverslips in a 6-well plate, and then the cells were treated with DMSO, cisplatin, or bleomycin, and extracted with extraction buffer (25 mM HEPES, pH 7.5, 50 mM NaCl, 1 mM EDTA, 3 mM MgCl2, 300 mM sucrose, and 0.5% Triton X-100). Thereafter, the cells were left on ice for 5 minutes and then fixed with 4% paraformaldehyde (PC2031-050-00, Biosesang) for 10 minutes. After washing three times with PBST (containing 0.1% Triton X-100 in PBS), the plate was blocked with a solution containing 10% FBS in PBST at room temperature for at least 30 minutes.

A primary antibody (anti-53BP1 antibody) was added and incubated with the cells for 2 hours, and then detection was performed for 40 minutes using a secondary antibody labeled with Alexa-555 (A21429; Life Technologies) or Alexa-488 (A11029; Life Technologies). The intracellular nuclei were stained with VECTASHIELD^{®} Antifade Mounting Medium containing DAPI (H-1200-10, Vector LABORATORIES). Images were captured using an Axio Imager.M2 microscope (Zeiss) and adjusted using ZEN3.4 (Zeiss, blue edition) and Photoshop 7.0 (Adobe).

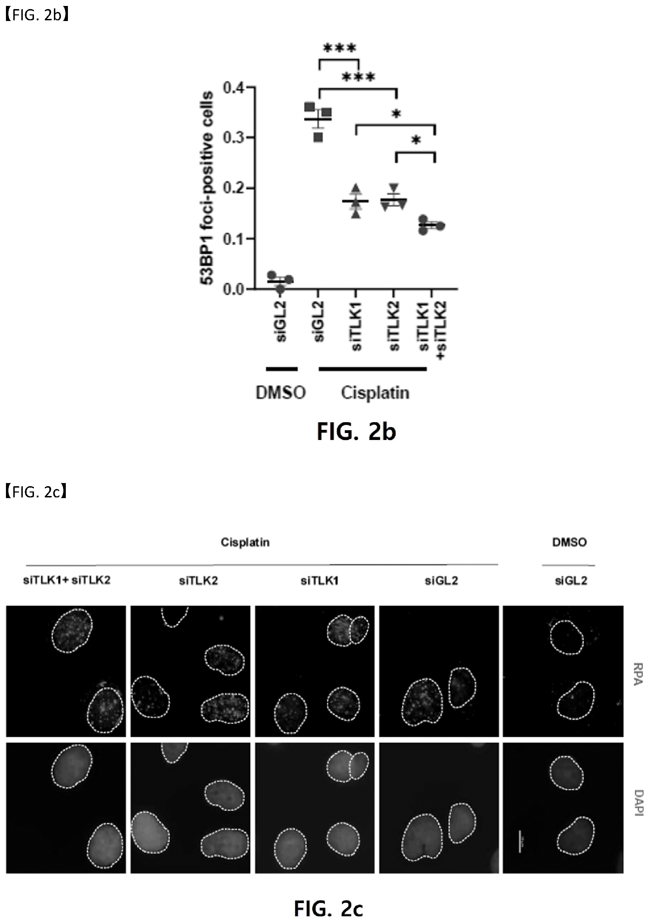

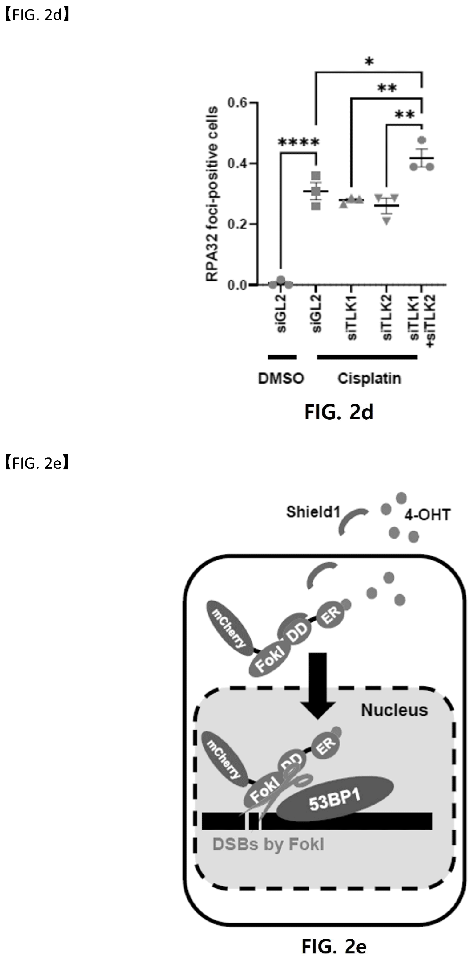

As a result, as expected, the number of 53BP1 focus-positive cells was decreased in cells in which TLK1 or TLK2 was depleted, and co-depletion of TLK1 and TLK2 caused a statistically significant further decrease (FIG. 2a and FIG. 2b). In particular, RPA32 foci, an indicator of ssDNA formation, were not decreased by depletion of TLK1 or TLK2, and interestingly, co-depletion increased the number of anti-RPA32-positive cells (FIG. 2c).

To confirm this, as shown in the schematic diagram of FIG. 2e, a single DSB was introduced at a defined site using U2OS ER-mCherry-Fokl-DD cells (Shanbhag, N.M., et al., Cell, 141:970-981, 2010; Tang, J., et al., Nat Struct Mol Biol, 20:317-325, 2013) treated with Shield-1 and 4-OHT. Shield-1 stabilizes Fokl by binding to the degron, whereas 4-OHT binds to the ER fused to Fokl to allow the protein to translocate into the nucleus. When cells are treated with 4-OHT and Shield-1, the mCherry-linked Fokl nuclease can bind to a specific Fokl cleavage site, and the localization of a protein of interest at the Fokl cleavage can be visualized through the mCherry signal.

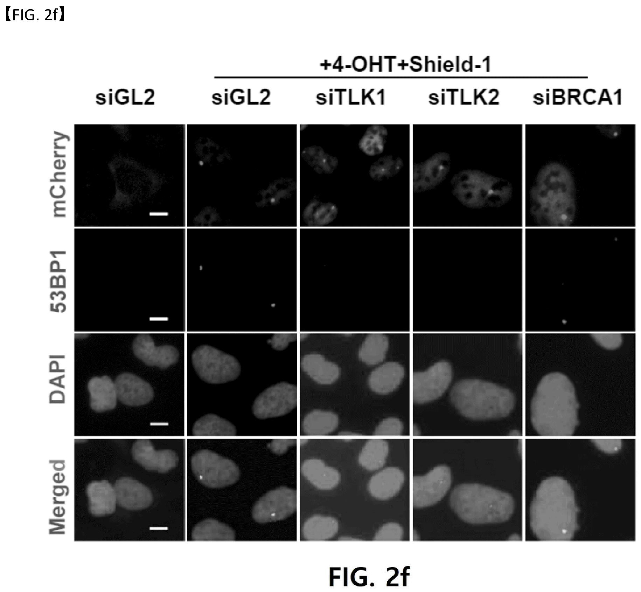

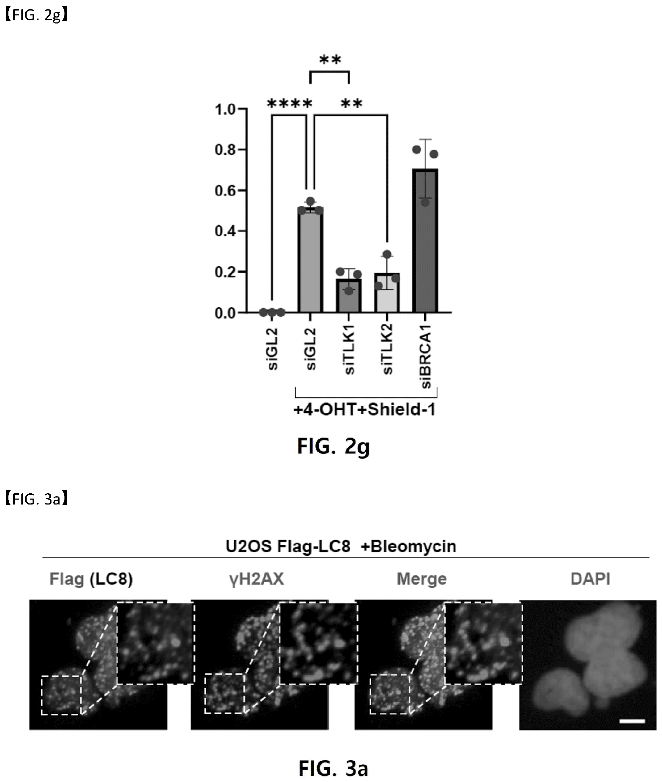

Depletion of TLK1 or TLK2 significantly decreased the number of cells having 53BP1 at the Fokl site, but depletion of BRCA1 as a negative control did not decrease the number (FIG. 2f and FIG. 2g). Therefore, through multiple experimental systems, the present invention confirmed that TLK1 and TLK2 are essential for the proper localization of 53BP1 at DSBs.

### Example 3: Confirmation of the DSB Localization of LC8 and Its Interaction with 53BP1 According to TLK Depletion

### 3-1: Confirmation of LC8 Localization According to TLK Depletion

TLK is known to promote NHEJ by enhancing the DSB localization of 53BP1 through interaction with several proteins, including LC8 (also known as DYNLL1, dynein light chain 1), which serves as a hub protein mediating the oligomerization of target proteins including 53BP1 (Kim, J.A., et al., Mol Cancer Res, 14:920-927, 2016). Since 53BP1 localization at DSBs is decreased when LC8 or TLK is depleted, the present invention sought to confirm whether the role of TLK protein in the activation of 53BP1 for NHEJ is exerted through LC8.

To visualize the DSB localization of LC8, the present invention prepared a U2OS cell line stably expressing Flag-LC8. LC8 cDNA was synthesized (Bioneer) and cloned into the pCAG-F-BSD (pCAG-FLAG-blasticidin) vector expressing an N-terminal FLAG tag. The plasmid was transfected into U2OS or U2OS EJ5 cells using Lipofectamine 2000 (11668019, Invitrogen) to generate a stable cell line, and then, 48 hours later, the cells were treated with 500 µg/mL G418 (Invivogen) for at least one week for drug selection.

Then, the cells were transfected with siRNA by the same method as in <Example 1>, and then treated with 2 µg/mL bleomycin (13877, Cayman Chemical) for 96 hours. Thereafter, immunostaining and immunoblotting were performed.

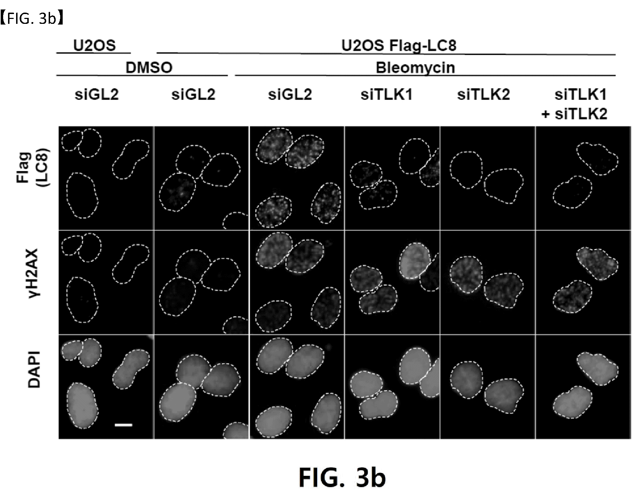

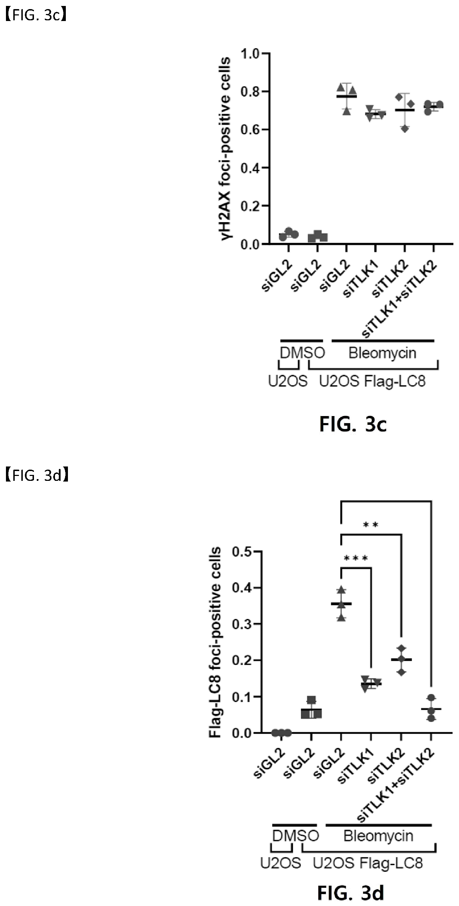

As a result, it was confirmed that the bleomycin-induced foci of Flag-LC8 almost overlaid with yH2AX foci, which means that Flag-LC8 is localized at DSBs (FIG. 3a). When TLK1 and/or TLK2 was depleted, the protein amount and focus formation of γH2AX did not decrease in bleomycin-treated cells, but the bleomycin-induced focus formation of Flag-LC8 was significantly suppressed in TLK1- and TLK2-depleted cells (FIG. 3b to FIG. 3d).

### 3-2: Confirmation of LC8 Through Subcellular Fractionation Analysis

To confirm the above results, the present invention performed a subcellular fractionation analysis to identify chromatin-associated LC8 in bleomycin-treated cells.

The subcellular fractionation assay was performed using a subcellular protein fractionation kit for cultured cells (78840, Thermo Scientific). U2OS cells were transfected with siRNA by the same method as in <Example 1>, and then treated with bleomycin.

After harvesting the cells and washing with cold PBS, half of the cells were lysed using 50 to 100 µL of RIPA buffer containing protease inhibitors to obtain a total cell extract. The remaining half of the cells was subjected to a sequential fractionation process.

Specifically, after incubation with 50 to 100 µL of CEB buffer containing protease inhibitors on ice for 10 minutes, the mixture was centrifuged at 500 g for 5 minutes to obtain a cytoplasmic extract. The remaining pellet was incubated with 25 to 50 µL of NEB buffer containing protease inhibitors on ice for 10 minutes to obtain a nuclear extract.

In the case of a chromatin extract, 25 to 50 µL of NEB containing MN enzyme supplemented with CaCl2 and protease inhibitors was incubated at 37°C for 5 minutes, and then centrifuged at 16,000 g for 5 minutes. Then, proteins were isolated from each fraction, and immunoblotting was performed. The immunoblots for histone H3, NBS1, and GAPDH were used as fractionation markers for chromatin, nucleosol, and cytoplasm, respectively. The immunoblots of yH2AX and histone H3 indicate the DNA damage response induced by bleomycin and chromatin enrichment of the extract, respectively; TCE denotes total cell extract, and Chr denotes the chromatin-enriched fraction.

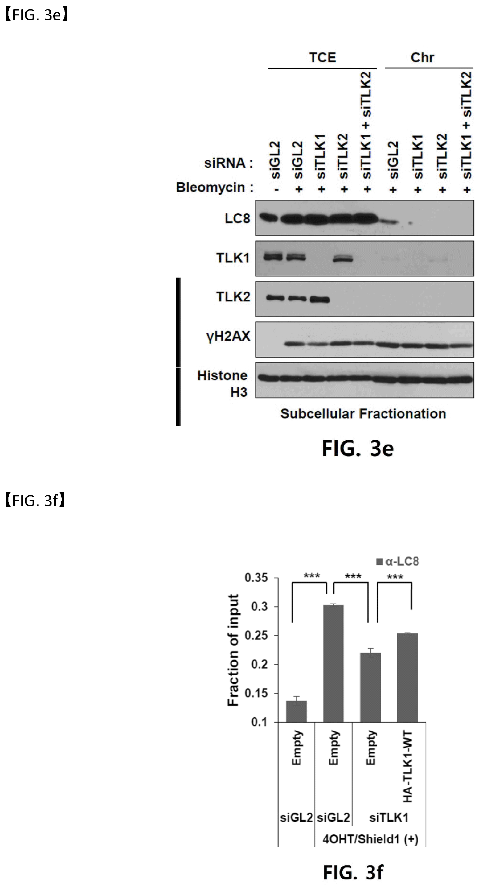

As a result, it was confirmed that endogenous LC8 of the chromatin fraction (Chr) is removed after TLK depletion (FIG. 3e).

### 3-3: Confirmation of LC8 Localization Through qChIP Analysis

In addition, since the chromatin association of LC8 may be at a site without a DSB, the present invention performed a ChIP analysis to measure the recruitment of LC8 to a DSB generated by Fokl nuclease in U2OS ER-mCherry-Fokl-DD cells.

In addition, in order to confirm the degree of restoration of LC8 localization at the Fokl cleavage site by siRNA-resistant TLK1 in TLK1-depleted cells, the cells were transfected using the siRNA-resistant TLK1-wild type (HA-TLK1-WT) of <Example 1-2>.

To perform a chromatin immunoprecipitation (ChIP) assay, U2OS ER-mCherry-Lacl-Fokl-DD cells transfected with HA-TLK1-WT or siRNA TLK were fixed with 1% formaldehyde at room temperature for 20 minutes and quenched by adding 1.5 M glycine for 5 minutes. The fixed cells were lysed with SDS lysis buffer (50 mM Tris-HCl, pH 8.0, 10 mM EDTA, pH 8.0, 0.1% SDS, and protease inhibitor cocktail (Roche)) on ice for 15 minutes, or sonicated using Covaris ultrasonication (P, 140; DF, 10; cycle, 200; treatment, 80 seconds at 4°C).

The resulting lysate was diluted with dilution buffer (50 mM Tris-HCl, pH 8.0, 150 mM NaCl, 1% Triton X-100, 0.1% SDS, and protease inhibitor cocktail) and incubated overnight with Protein G beads conjugated to dyna-beads bound with an anti-LC8 antibody. The beads were washed sequentially with RIPA-150 buffer (50 mM Tris-HCl, pH 8.0, 150 mM NaCl, 1 mM EDTA, pH 8.0, 1% Triton X-100, 0.1% SDS, and 0.1% sodium deoxycholate), LiCl buffer (10 mM Tris-HCl, pH 8.0, 0.25 M LiCl, 1 mM EDTA, pH 8.0, 0.5% NP40, and 0.5% sodium deoxycholate), and TE buffer.

Then, the beads were incubated with ChIP elution buffer (10 mM Tris-HCl, pH 8.0, 300 mM NaCl, 5 mM EDTA, pH 8.0, and 0.5% SDS) at 65°C for 8 hours, after which the IPed DNA was recovered using a QIAquick PCR Purification Kit (Qiagen), and qPCR analysis was performed using a LightCycler 96 (Roche). The primer sequences used in the qPCR analysis are as follows:

### <Primer Sequences>

F: 5'-GGCATTTCAGTCAGTTGCTCAA-3' (SEQ ID NO: 12)
R: 5'-TTGGCCGATTCATTAATGCA-3' (SEQ ID NO: 13)

As a result, in TLK1-depleted cells, the enrichment of LC8 at the DSB was suppressed but was partially restored by siRNA-resistant HA-TLK1 WT (FIG. 3f). These data show that, in particular, the chromatin binding of LC8 at the DSB depends on the presence or absence of TLK in the cell, and that LC8 plays a role in DSB localization and the oligomerization of 53BP1 through physical interaction.

### 3-4: Confirmation of the Interaction Between 53BP1 and LC8 Upon TLK Depletion

In the present invention, it was verified whether TLK is required for the physical interaction between LC8 and 53BP1.

H1299 cells were cultured in RPMI 1640 (Corning) supplemented with 10% FBS and 1% penicillin/streptomycin, transfected with siRNA by the same method as in <Example 1>, and then treated with bleomycin.

For immunoprecipitation, H1299 cells were lysed in lysis buffer (20 mM Tris-HCl, pH 8.0, 150 mM NaCl, 1% NP-40, 2 mM EDTA, phosphatase inhibitors, and protease inhibitors) for 15 minutes on ice. Then, the lysate was briefly sonicated and centrifuged at 15,000 rpm for 30 minutes at 4°C. Approximately 1 to 1.5 mg of the lysate was incubated with an anti-53BP1 antibody or EZview Red Anti-HA Affinity Gel (E6779, Sigma), and then the immunoprecipitated 53BP1 was pulled down using Protein A Sepharose beads (GE Healthcare). After washing the beads or gel five times with lysis buffer, immunoblotting was performed.

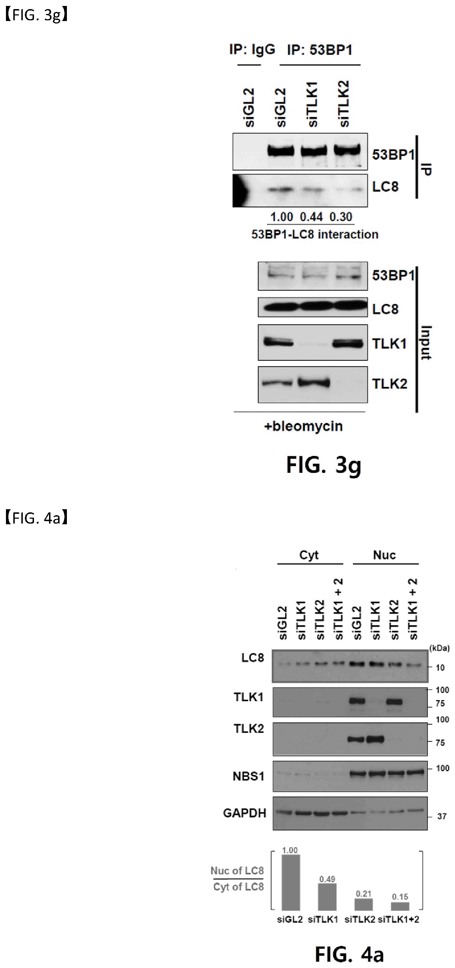

As a result, the 53BP1 immunoprecipitate contained LC8 in normal cells (siGL2), but when TLK1 or TLK2 was depleted, the amount of co-immunoprecipitated LC8 was decreased upon bleomycin treatment (FIG. 3g), suggesting that TLK is required for the 53BP1-LC8 interaction. That is, the above results support the concept that TLK1 and TLK2 promote the DSB localization of LC8 by promoting its interaction with 53BP1.

### Example 4: Confirmation of the Nuclear Translocation of LC8 and NHEJ by the Nuclear Localization Signal (NLS) of TLK

### 4-1: Confirmation of LC8 Distribution According to TLK Depletion

In the present invention, the manner in which TLK induces the DSB localization of LC8 was investigated. The physical interaction between TLK and LC8 requires a coiled-coil domain (Segura-Bayona, S., et al., Cell Death Differ, 24:1872-1885, 2017). Despite the absence of a nuclear localization signal (NLS) sequence within LC8, since LC8 functions to regulate the multimerization of 53BP1 in the nucleus, the present invention hypothesized that the nuclear translocation of LC8 is mediated by its interaction with TLK.

Therefore, in the present invention, U2OS cells were transfected with siRNA and then treated with bleomycin, after which the nuclear and cytoplasmic distribution of LC8 in the case of TLK depletion was investigated using subcellular fractionation analysis by the same method as in <Example 3-2>.

As a result, when TLK1 or TLK2 was depleted, the amount of LC8 protein in the nucleus was decreased and the cytoplasmic distribution ratio was increased. In addition, it was confirmed that simultaneous depletion of TLK1 and TLK2 maximizes the depletion of LC8 in the nuclear fraction (FIG. 4a). The above results support the concept that TLK is required for the nuclear transport of LC8.

### 4-2: Analysis of the Nuclear Localization Signal (NLS) of TLK

In the present invention, it was sought to confirm whether the NLS of TLK is used for the nuclear translocation of LC8. The putative NLS sequence was predicted using the cNLS Mapper program available at http//nls-mapper.iab.keio.ac.jp.

The putative NLS sequence was predicted using the cNLS Mapper program available at http//nls-mapper.iab.keio.ac.jp, and COBALT (Constraint-based Alignment Tool) was used for this alignment (https://www.ncbi.nlm.nih.gov/tools/cobalt/cobalt.cgi?CMD=Web).

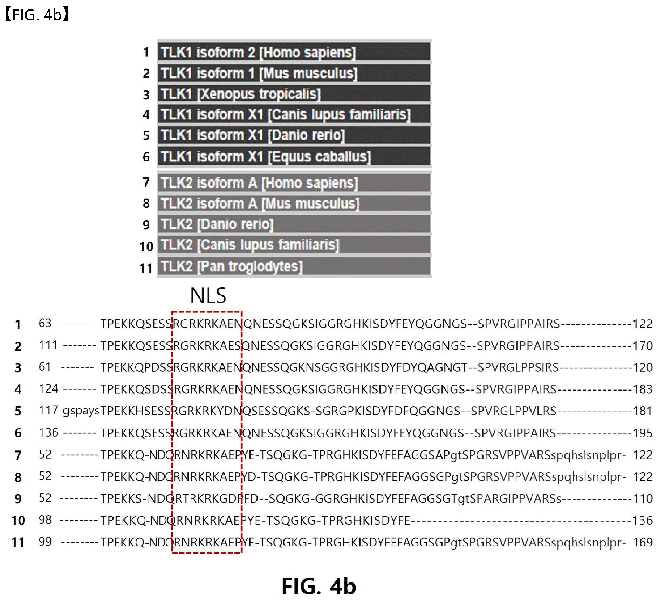

As a result, a single NLS motif was identified in TLK1, and it was found to be highly conserved not only in TLK2 but also across various species (FIG. 4b, SEQ ID NO: 17 to SEQ ID NO: 27).

### 4-3: Confirmation of the Interaction with LC8 According to NLS Mutation of TLK

In addition, in the present invention, a nuclear localization-defective mutant of HA-TLK1 (NLSm) (RGRAAKAENQ, SEQ ID NO: 16) was prepared by substituting KR (RGRKRKAENQ, SEQ ID NO: 15) with AA in the NLS 9 motif.

HA-TLK1 (NLSm) was generated by the same method as in <Example 1-2>, using the following primer:
TLK1 NLSm - F: 5'-GGAATCATCCAGGGGAAGAGCGGCAAAAGCAGAAAACCAGAATG-3' (SEQ ID NO: 14)

Then, HA-TLK1-WT or NLSm was overexpressed in HEK293T cells, after which immunostaining and immunoprecipitation using an anti-HA antibody were performed.

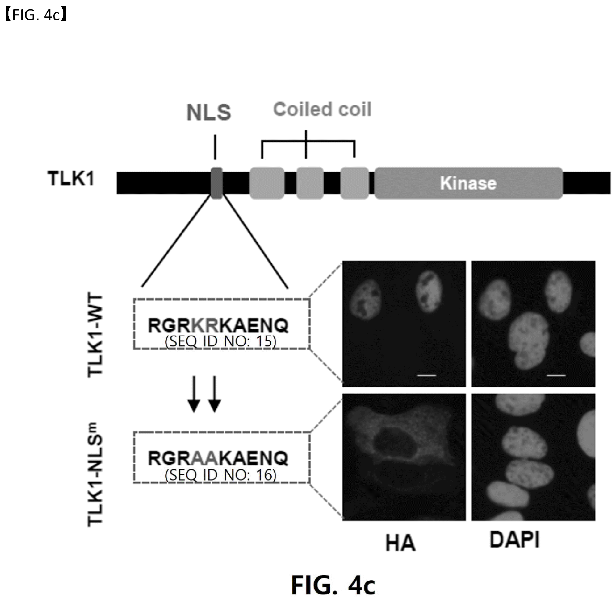

FIG. 4c is a schematic diagram of the functional motifs of TLK1 including the NLS; as a result of substituting amino acids that inactivate the NLS function of TLK1 and the cytoplasmic localization of HA-TLK1 NLSm, the nuclear localization of the HA-TLK1 NLS mutant failed, and the localization of TLK1 NLSm in the cytoplasm was observed.

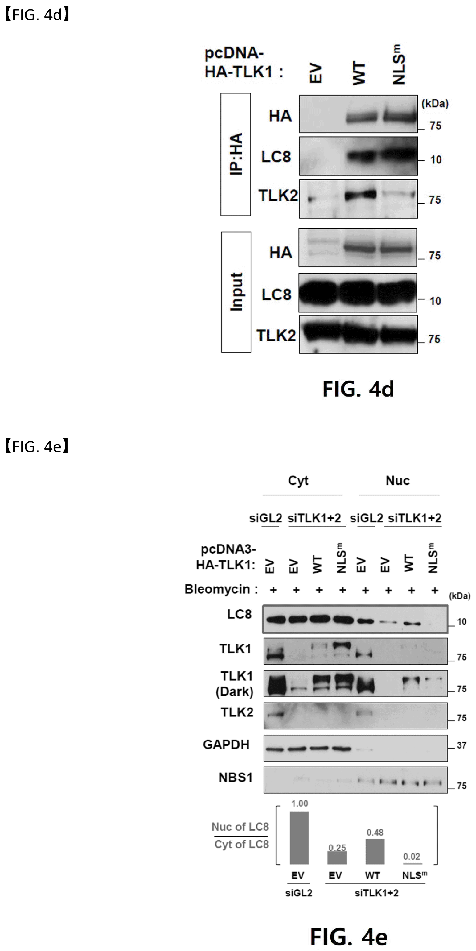

In addition, it was found that the HA-TLK1 NLSm immunoprecipitate contained the same LC8 as HA-TLK1 WT, which indicates that the interaction of TLK1 with LC8 is independent of subcellular localization (FIG. 4d).

In the present invention, it was confirmed that HA-TLK1 NLSm fails to interact with endogenous TLK2, suggesting that the nuclear localization of TLK acts differently by interacting with LC8 and TLK oligomerization.

### 4-4: Confirmation of 53BP1-Dependent NHEJ Promotion According to the Nuclear Localization of TLK

Next, in the present invention, it was evaluated whether the nuclear localization of TLK is important for the nuclear translocation of LC8, and whether it thereby contributes to the promotion of 53BP1-dependent NHEJ.

U2OS EJ5 reporter cells stably expressing siRNA-resistant HA-TLK1-WT, HA-TLK1-KD, or -NLSm were transfected with siRNA by the same method as in <Example 1>, and then treated with bleomycin. Thereafter, subcellular fractionation analysis and NHEJ analysis were performed.

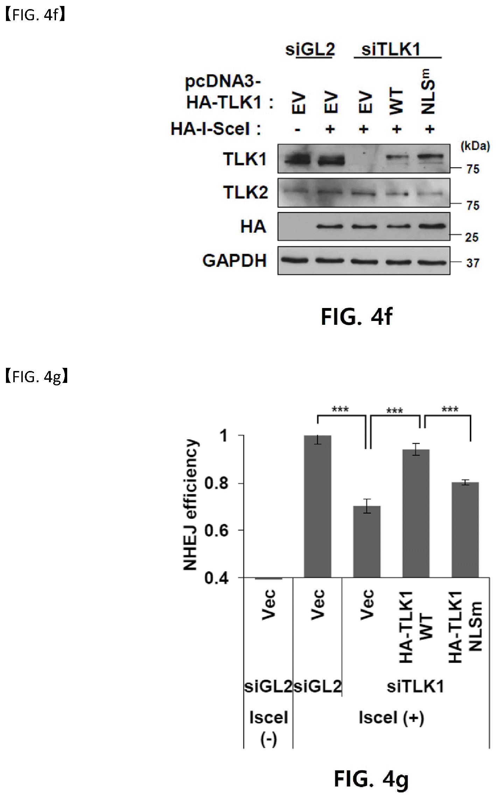

As a result, it was confirmed that the decrease in LC8 in the nuclear fraction after depletion of TLK1 and TLK2 was successfully restored by siRNA-resistant HA-TLK1 WT but was not restored in NLSm (FIG. 4e). Since the binding of LC8 to NLSm was not decreased compared to the binding to WT, the above result can be attributed to the fact that most of the LC8 that interacts with NLSm is present in the cytoplasm. In addition, upon TLK1 depletion, the decrease in NHEJ efficiency was recovered by WT but was not recovered by NLSm (FIG. 4f and FIG. 4g).

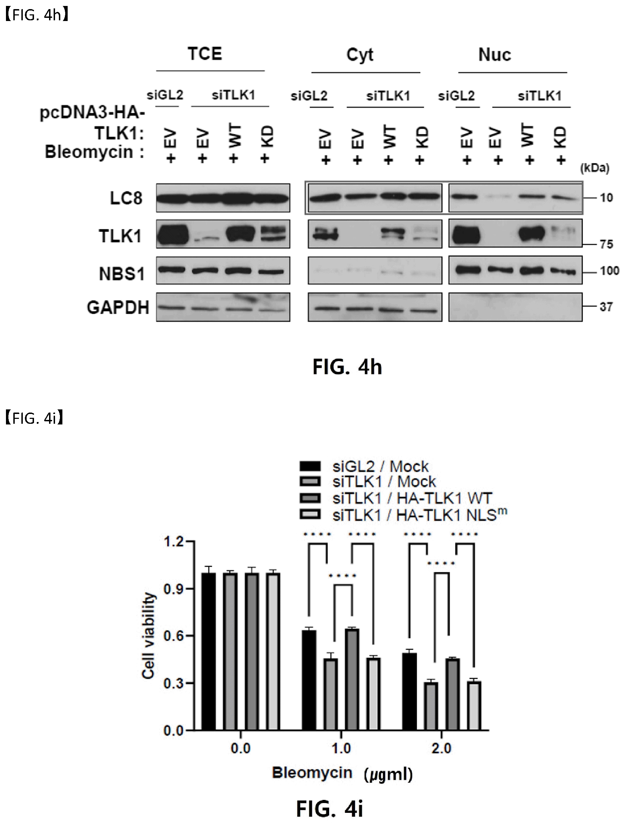

Furthermore, TLK1-KD restored the nuclear translocation of LC8 upon TLK depletion, which means that TLK promotes the nuclear translocation of LC8 in a kinase-independent manner (FIG. 4h).

Finally, in the present invention, it was found that NLSm failed to alleviate the cellular sensitivity to bleomycin observed upon TLK depletion (FIG. 4i).

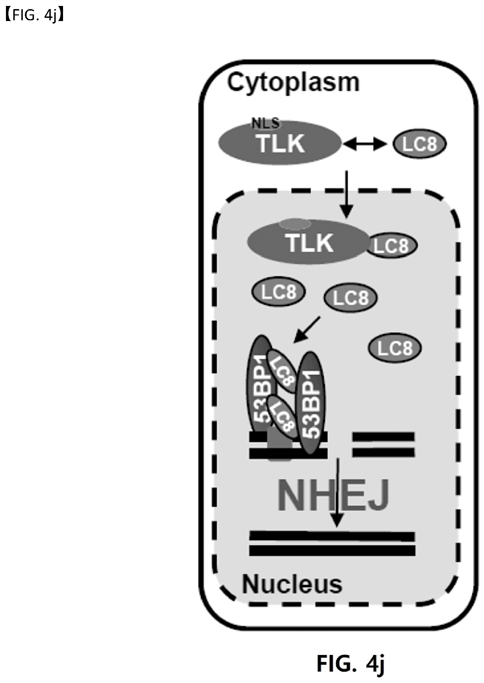

Taken together, the above results support the conclusion that, as shown in the schematic diagram of FIG. 4j, the NLS of TLK promotes 53BP1-dependent NHEJ by mediating the nuclear translocation of LC8.

### Example 5: Confirmation of PARP Inhibitor Resistance in BRCA1 Mutant Cancer According to TLK Depletion

The loss of NHEJ is known to render BRCA1 mutant cells resistant to PARP inhibitors by restoring a certain level of HR. Therefore, in the present invention, it was investigated whether TLK depletion can confer resistance to PARP inhibitors.

Immunoblot analysis was performed on each of BRCA1-proficient MDA-MB-468 breast cancer cells (BRCA1^{WT}) and BRCA1-mutant MDA-MB-436 cells (BRCA1^{m}), and after treatment with olaparib or veliparib as PARP inhibitors at various concentrations, cell viability analysis was performed.

For the depletion of TLK1 or TLK2, the same siRNA as in <Example 1-1> was used, and siBRCA1 was used to confirm the effect of BRCA1 depletion in BRCA1-proficient MDA-MB-468 cells.

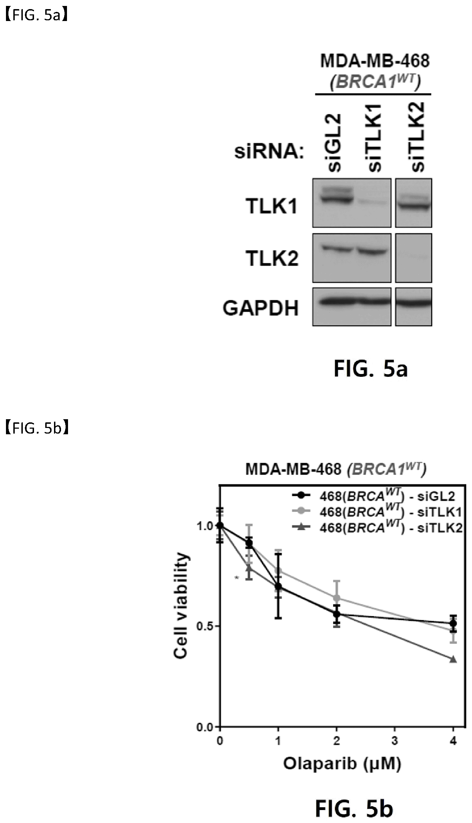

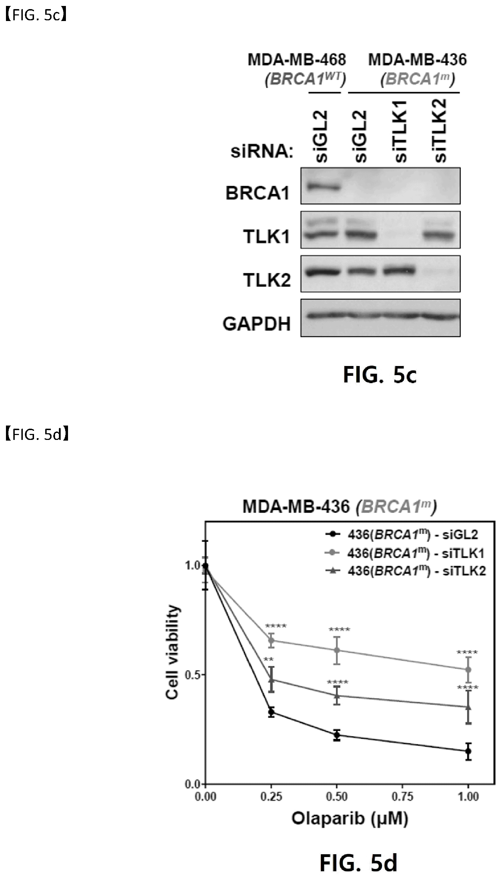

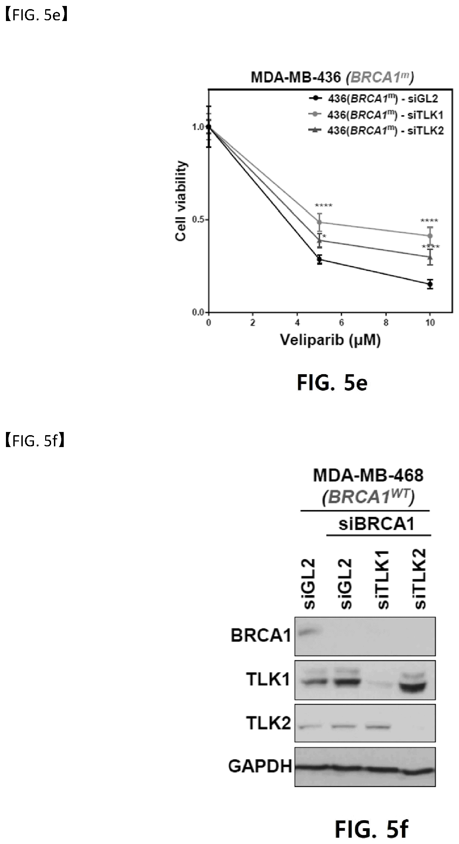

As a result, in MDA-MB-468 cells, which are BRCA1-proficient triple-negative breast cancer (TNBC) cells, depletion of TLK1 or TLK2 did not cause any change in PARP inhibitor sensitivity in the cell viability analysis using olaparib (FIG. 5a and FIG. 5b).

However, in MDA-MB-436 cells having a BRCA1 mutation, when TLK1 or TLK2 was depleted, resistance to the PARP inhibitors olaparib and veliparib was increased (FIG. 5c to FIG. 5e).

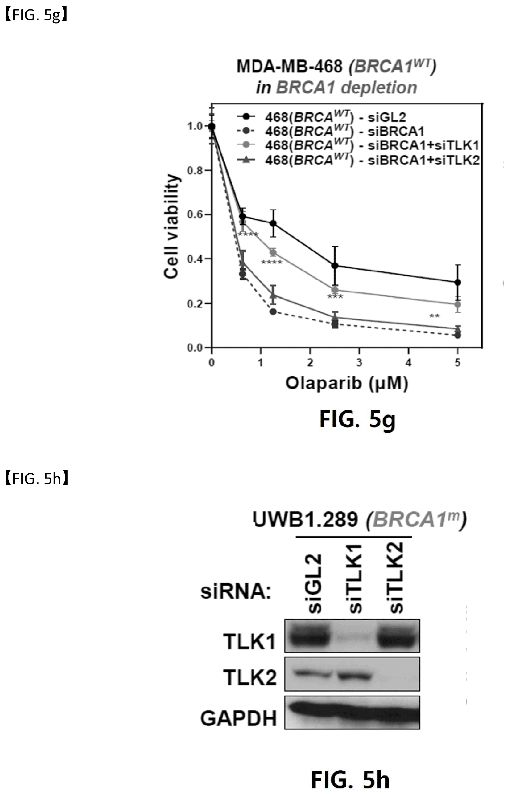

As expected, in MDA-MB-468 cells, which are BRCA1-proficient triple-negative breast cancer cells, when BRCA1 was depleted by siRNA BRCA1, the cells became sensitive to olaparib, but it was confirmed that when BRCA1 and TLK were simultaneously depleted, the cells exhibited resistance to olaparib (FIG. 5f and FIG. 5g).

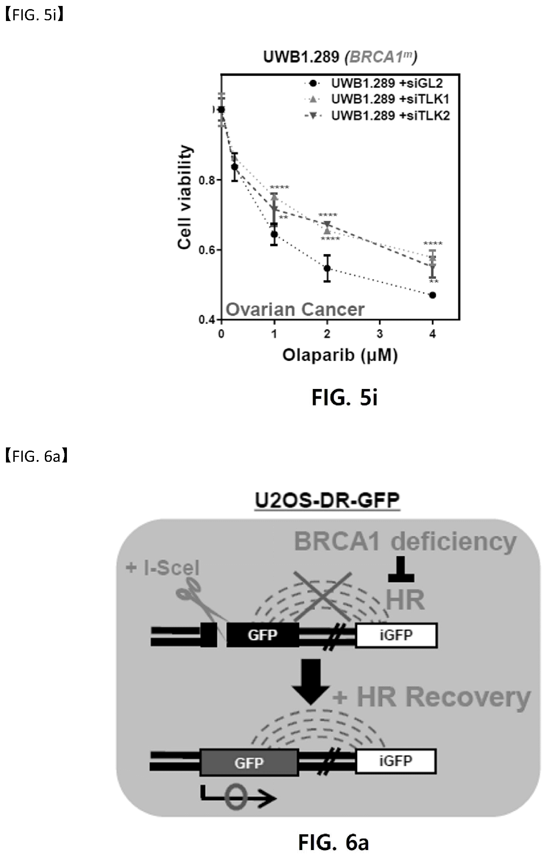

In addition, UWB1.289, a BRCA1-mutant ovarian cancer cell line, was shown to have decreased sensitivity to olaparib upon depletion of TLK1 or TLK2 (FIG. 5h and FIG. 5i).

The results of the present invention mean that TLK expression is functionally proportional to PARP inhibitor sensitivity in BRCA1 mutant cells.

### Example 6: Confirmation of HR Restoration in BRCA1 Mutant Cells According to TLK Depletion

### 6-1: Confirmation of HR Restoration According to TLK Depletion

Because BRCA1 is essential for HR and HR deficiency promotes the toxicity of PARP inhibitors, the efficacy of PARP inhibitors is maximized in BRCA1-mutant, HR-deficient cancer. However, when NHEJ is inactivated in HR deficiency (HRD), the nucleases required for HR can partially restore HR, which is an important mechanism for resistance to PARP inhibitors (Lord, C.J., Tutt, A.N., and Ashworth, A, Annu Rev Med 66, 455-470, 2015; Jaspers, J.E., et al., Cancer Discov 3, 68-81, 2013).

Since the present invention confirmed that TLK depletion promotes resistance to PARP inhibitors, it was confirmed whether the increased resistance to PARP inhibitors was due to HR being recovered in BRCA1-deficient cells when TLK1 was depleted.

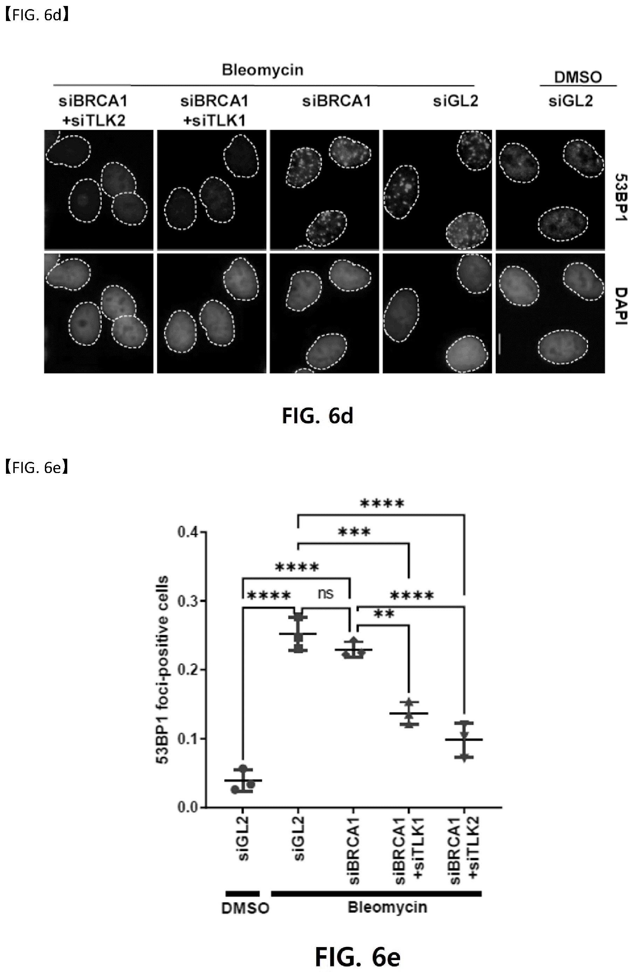

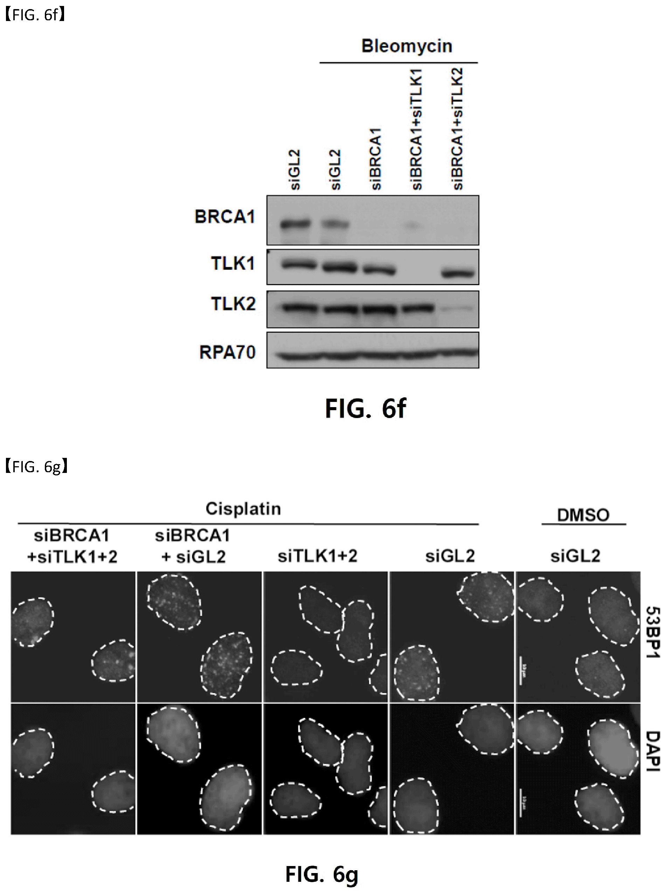

FIG. 6a is a schematic diagram illustrating the measurement of HR efficiency according to BRCA1 depletion in U2OS DR-GFP cells, in which immunoblot analysis and HR efficiency were confirmed when U2OS DR-GFP cells were transfected with siBRCA1 and siRNA TLK.

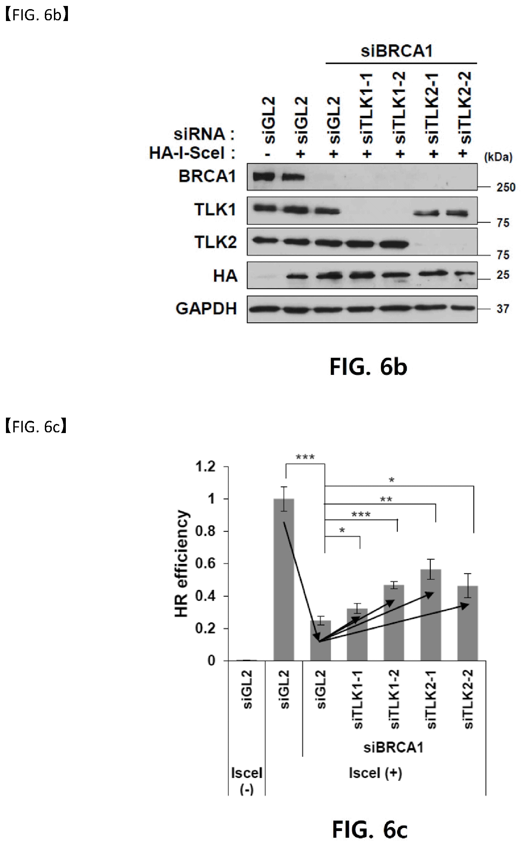

As a result of analyzing the efficiency of HR using the U2OS DR-GFP cell line, the HR efficiency was decreased to 25% upon BRCA1 depletion. However, when TLK1 or TLK2 was co-depleted in BRCA1-depleted cells, a significant amount of HR was recovered, and the HR efficiency was shown to be about 55% that of wild-type cells (FIG. 6b and FIG. 6c). That is, it was confirmed that HR is restored by depletion of TLK1 or TLK2, rather than by BRCA1 depletion.

### 6-2: Observation of 53BP1 Focus-Positive Cells According to TLK Depletion

Next, it was investigated whether the HR restoration upon TLK depletion is due to NHEJ impairment resulting from 53BP1 not being localized at DSBs.

When A549 cells were transfected with siBRCA1 and siRNA TLK and then treated with bleomycin or cisplatin, 53BP1 focus-positive cells were observed by immunostaining.

As a result, after co-depletion of TLK1 or TLK2 together with BRCA1, 53BP1 localization-positive cells were decreased, which indicates that TLK is essential for 53BP1 binding to DSBs even in BRCA1-deficient cells (FIG. 6d to FIG. 6f).

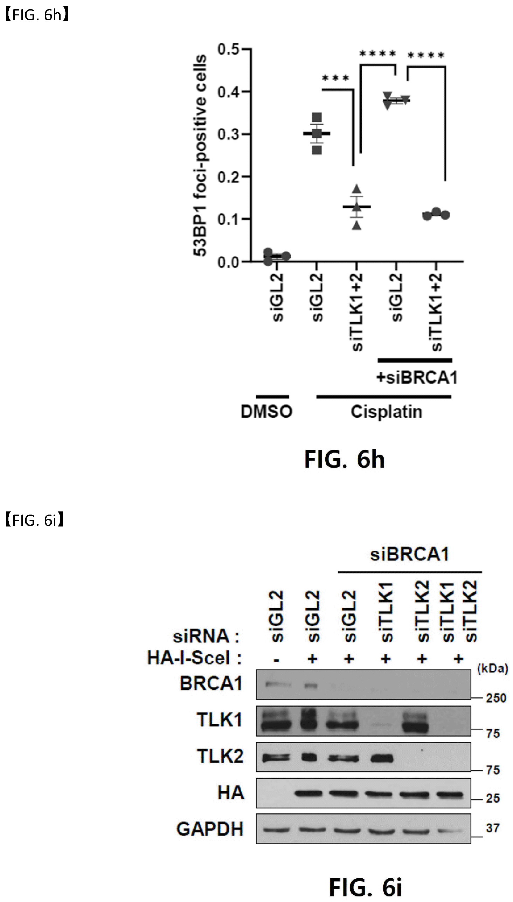

In addition, the decrease in 53BP1 localization-positive cells after depletion of TLK1 and TLK2 was the same regardless of whether the cells were free of or deficient in a BRCA1 mutation, which indicates that the importance of TLK in 53BP1 binding to DSBs does not depend on the HR capacity of the cells (FIG. 6g and FIG. 6h).

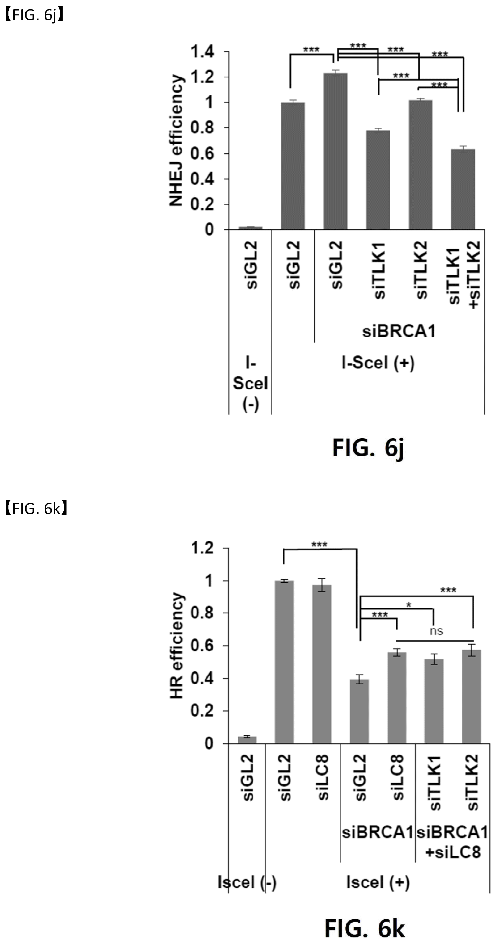

In the present invention, NHEJ efficiency was measured to confirm whether NHEJ was inactivated by TLK depletion in BRCA1-depleted cells (FIG. 6i and FIG. 6j).

As a result, BRCA1 depletion slightly increased NHEJ efficiency, but the increase in NHEJ in BRCA1-deficient cells was reversed by depletion of TLK1 and/or TLK2, which demonstrates that TLK is required for NHEJ repair even in HR-deficient cells (FIG. 6i and FIG. 6j).

### 6-3: Analysis of NHEJ Efficiency According to TLK Depletion

In the present invention, in order to confirm whether NHEJ was inactivated by TLK depletion in BRCA1-depleted cells, NHEJ/DsRed293B cells were transfected with siBRCA1 and siRNA TLK, and then the NHEJ efficiency was measured.

As a result, BRCA1 depletion slightly increased NHEJ efficiency, but the increase in NHEJ in BRCA1-deficient cells was reversed by depletion of TLK1 and/or TLK2, which demonstrates that TLK is required for NHEJ repair even in HR-deficient cells (FIG. 6i and FIG. 6j).

### 6-4: Confirmation that TLK and LC8 Act in the Same DSB Repair Pathway

LC8 reduction is known to increase HR efficiency in BRCA1-deficient cells, thereby inducing resistance to PARP inhibitors. Since LC8 is also required for forming 53BP1 foci, which is the same step that depends on TLK1, it was expected that co-depletion of TLK and LC8 would not further increase HR efficiency compared to single depletion of LC8.

In the present invention, in order to confirm whether TLK and LC8 act in the same DSB repair pathway upon BRCA1 inactivation, HR analysis was performed using the method of FIG. 6a, in which U2OS DR-GFP cells were transfected with siBRCA1, siLC8, or siRNA TLK of <Example 1-1>, and then the HR efficiency was analyzed.

As a result, it was confirmed that TLK and LC8 act in the same pathway for DSB repair under the BRCA1-deficient condition (FIG. 6k).

### Example 7: Observation of Prognosis According to TLK Depletion in BRCA1-Deficient Patients

LC8 expression provides a better prognosis and sensitivity to PARP inhibitors in BRCA1 mutant cancer. In the present invention, it was confirmed that BRCA1-deficient cells exhibit resistance to PARP inhibitors upon TLK depletion. According to some studies, TLK has been shown to be increased in certain cancers with a poor prognosis. That is, unlike the result of the present invention in which cancer acquires PARP inhibitor resistance upon TLK depletion, previous research results have shown that TLK loss predicts a better prognosis. Of course, the survival studies on TLK were conducted on patients who were not treated with PARP inhibitors.

However, due to the interaction between BRCA1 status and TLK level in the present invention, there is another possibility, in that the survival effect of the TLK level may differ between BRCA1-proficient tumors and BRCA1-deficient tumors even when the tumors are not treated with PARP inhibitors.

To confirm this, in the present invention, tumors of the TCGA database were analyzed to investigate whether the correlation between TLK expression and patient survival is affected by BRCA1 status.

Since a sufficient number of BRCA1-deficient patients cannot be utilized for some cancers, the following nine cancers were analyzed: Head and Neck Squamous Cell Carcinoma (HNSC), Colon adenocarcinoma (COAD), Kidney renal clear cell carcinoma (KIRC), Uterine Corpus Endometrial Carcinoma (UCEC), Ovarian cancer (OVCA), Breast invasive carcinoma (BRCA), Cervical squamous cell carcinoma and endocervical adenocarcinoma (CESC), Bladder Urothelial Carcinoma (BLCA), and Lung adenocarcinoma (LUAD).

All TCGA data were downloaded via the TCGAbiolinks package in R and prepared according to the package manual. BRCA1 was considered "mutated" in the following cases:
a) recorded as containing one or more single nucleotide variants (SNVs);
b) copy number less than 2;
c) expression z-score less than 2.

In addition, in the case of BRCA and OVCA, patients with a germline mutation in BRCA1 were also considered mutated. Each cancer was initially classified into two cohorts according to the mutation status of BRCA1, and further analysis was performed only when both cohorts contained 20 or more cases. Then, each cohort was classified into two groups according to the degree of TLK expression, using a minimum sample number of 10 and a threshold that yields the lowest p-value in the log-rank test of the Kaplan-Meier analysis. Nine cancers satisfied the aforementioned criteria: BLCA, BRCA, CESC, COAD, HNSC, KIRC, LUAD, OVCA, and UCEC.

To calculate the regression coefficients, Cox proportional hazards regression analysis was performed, which represents the hazard probability of the low-expression group relative to the high-expression group. A positive value indicates a high risk, and a negative value indicates a low risk. All data analyses were performed using R version 4.2.1.

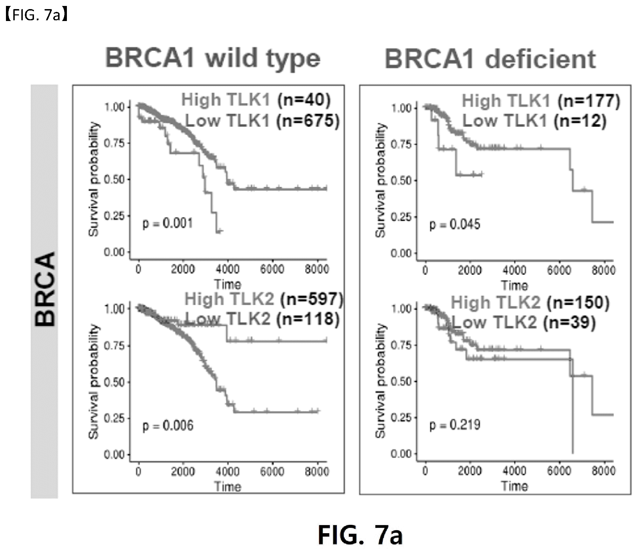

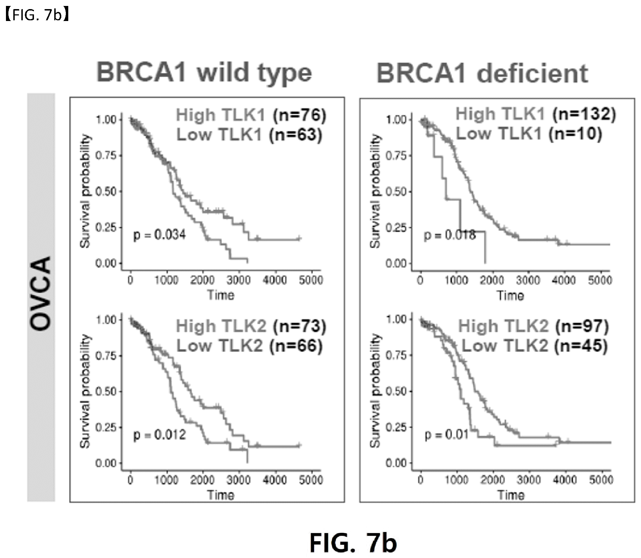

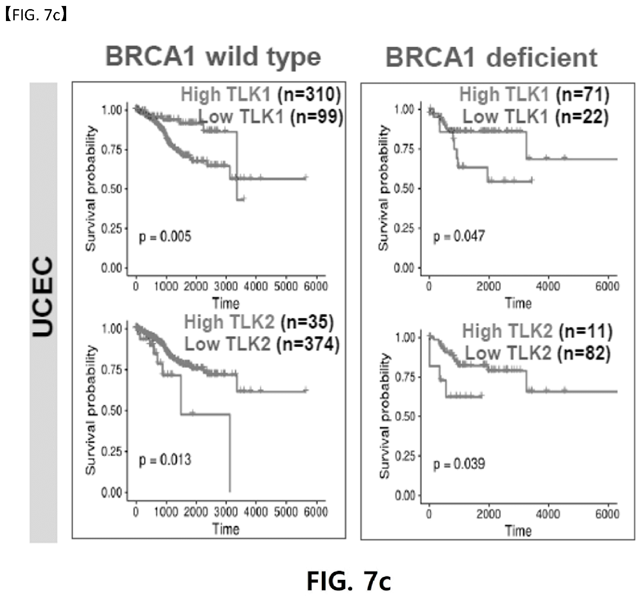

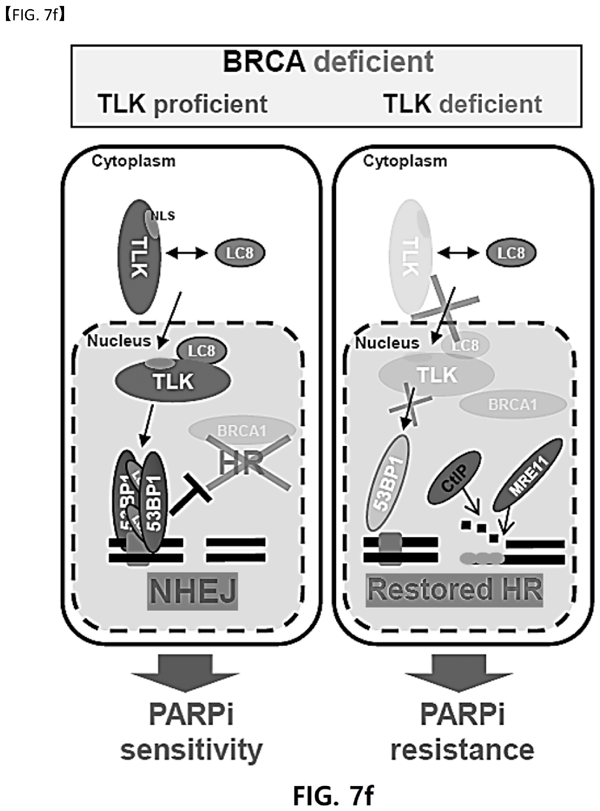

As a result, BRCA1-proficient cancer patients with BRCA, OVCA, and UCEC had poor outcomes when the degree of TLK1 or TLK2 expression was elevated, whereas BRCA1 mutant cancer patients were observed to have a better prognosis when the degree of TLK1 or TLK2 expression was elevated (FIG. 7a to FIG. 7c).

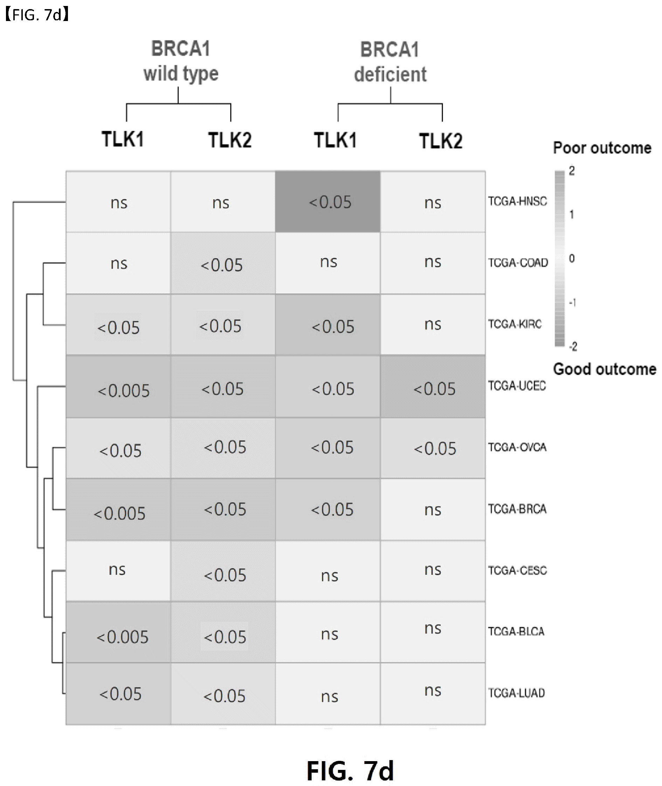

In an extensive investigation of multiple cancers, high expression of TLK1 or TLK2 was significantly associated with a poor prognosis in most cancer types having wild-type BRCA1 (or low TLK1 associated with a good outcome), but this phenomenon did not appear in tumors having BRCA1 deficiency (FIG. 7d).

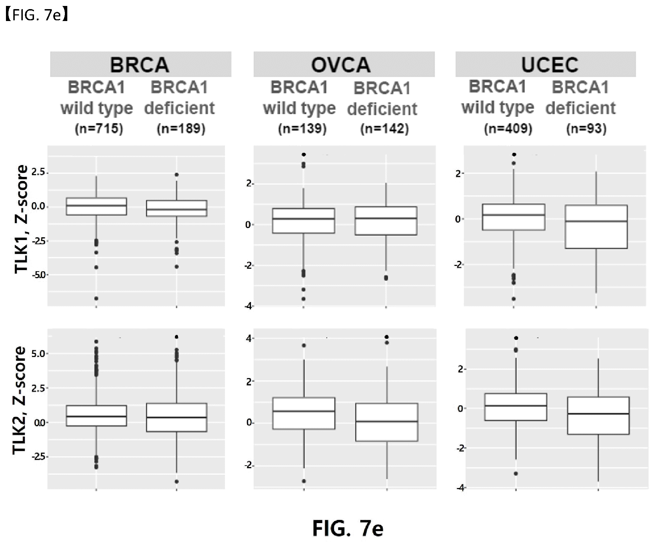

In addition, while TLK1 expression was significantly lower in BRCA and UCEC, which are BRCA1 mutant cancers, TLK2 expression was confirmed to be lower in OVCA and UCEC, which are BRCA1 mutant cancers, compared to BRCA1-proficient cancers (FIG. 7e). This result means that, when a tumor is BRCA1-deficient, the tumor may select for decreased TLK expression even before treatment with a PARP inhibitor. That is, regardless of PARP inhibitor treatment, a low TLK level can promote tumorigenesis in BRCA1 mutant cancer.

In the present invention, it was confirmed that, in cancer cells having a BRCA1 mutation, human TLK (Tousled-like Kinase) is essential for NHEJ-mediated DSB repair and PARP inhibitor sensitivity; therefore, the TLK biomarker of the present invention can be usefully applied to predicting the treatment responsiveness to, or prognosis of, a PARP inhibitor in BRCA mutant cancer.

## Claims

1. A TLK (Tousled-like kinase) biomarker composition for predicting the treatment responsiveness to, or prognosis of, a PARP inhibitor in BRCA mutant cancer.

2. The biomarker composition of claim 1, wherein the TLK biomarker is TLK1 or TLK2, and the PARP inhibitor is olaparib, talazoparib, niraparib, rucaparib, veliparib, or pamiparib.

3. The biomarker composition of claim 1, wherein the BRCA mutant cancer is breast cancer, ovarian cancer, or uterine cancer in which a BRCA mutation is present.

4. A composition for predicting the treatment responsiveness to, or prognosis of, a PARP inhibitor in BRCA mutant cancer, comprising an agent for measuring the mRNA or protein expression level of TLK (Tousled-like kinase).

5. The composition for predicting the treatment responsiveness to, or prognosis of, a PARP inhibitor in BRCA mutant cancer of claim 4, wherein the TLK biomarker is TLK1 or TLK2, and the PARP inhibitor is olaparib, talazoparib, niraparib, rucaparib, veliparib, or pamiparib.

6. The composition for predicting the treatment responsiveness to, or prognosis of, a PARP inhibitor in BRCA mutant cancer of claim 4, wherein the agent for measuring the mRNA expression level is a sense and antisense primer, or a probe, that complementarily binds to the mRNA of TLK, and the agent for measuring the protein expression level is an antibody, an interacting protein, a ligand, a nanoparticle, or an aptamer that specifically binds to the TLK protein or peptide fragment.

7. The composition for predicting the treatment responsiveness to, or prognosis of, a PARP inhibitor in BRCA mutant cancer of claim 4, wherein the BRCA mutant cancer is breast cancer, ovarian cancer, or uterine cancer in which a BRCA mutation is present.

8. A method of providing information on the treatment responsiveness to, or prognosis of, a PARP inhibitor in BRCA mutant cancer, comprising the step of measuring the mRNA or protein expression level of TLK from a sample of a BRCA mutant cancer patient.

9. The method of providing information on the treatment responsiveness to, or prognosis of, a PARP inhibitor in BRCA mutant cancer of claim 8, wherein, when the mRNA or protein expression level of TLK is lower than that of a normal control, information is provided indicating that the treatment responsiveness to the PARP inhibitor is low or that the prognosis is poor.

10. A method of providing information on the administration of a PARP inhibitor in BRCA mutant cancer, comprising the step of measuring the mRNA or protein expression level of TLK from a sample of a BRCA mutant cancer patient.

11. The method of providing information on the administration of a PARP inhibitor in BRCA mutant cancer of claim 10, wherein, when the mRNA or protein expression level of TLK is lower than that of a normal control, information is provided indicating that the PARP inhibitor is not to be administered.

12. A pharmaceutical composition for preventing or treating BRCA mutant cancer that exhibits resistance to a PARP inhibitor, comprising a substance for promoting the mRNA or protein expression level of TLK.

13. The pharmaceutical composition for preventing or treating BRCA mutant cancer that exhibits resistance to a PARP inhibitor of claim 12, wherein the composition further comprises a PARP inhibitor.

14. The pharmaceutical composition for preventing or treating BRCA mutant cancer that exhibits resistance to a PARP inhibitor of claim 12, wherein the PARP inhibitor is olaparib, talazoparib, niraparib, rucaparib, veliparib, or pamiparib.

15. A screening method for a substance for preventing or treating BRCA mutant cancer that exhibits resistance to a PARP inhibitor, comprising the steps of treating BRCA mutant cancer cells with a test substance and then measuring the mRNA or protein expression level of TLK.
